(19) Europäisches Patentamt
European Patent Office
Office européen des brevets

(11) Publication number: 0 260 149 A1

# (12) EUROPEAN PATENT APPLICATION

(21) Application number: 87308062.6

(22) Date of filing: 11.09.87

(51) Int. Cl.[3]: C 12 N 15/00
C 12 P 21/02, C 07 K 7/10
C 12 N 5/00, A 61 K 37/24

(30) Priority: 12.09.86 US 907197

(43) Date of publication of application:
16.03.88 Bulletin 88/11

(84) Designated Contracting States:
AT BE CH DE ES FR GB GR IT LI LU NL SE

(71) Applicant: HOWARD FLOREY INSTITUTE OF EXPERIMENTAL PHYSIOLOGY AND MEDICINE
c/o University of Melbourne
Parkville Victoria(AU)

(71) Applicant: GENENTECH, INC.
460 Point San Bruno Boulevard
South San Francisco California 94080(US)

(72) Inventor: Gorman, Cornelia Maxine
1122 Church Street
San Francisco California 94114(US)

(72) Inventor: Ross, Michael Jay
1065 Hayne Road
Hillsborough California 94010(US)

(72) Inventor: Niall, Hugh David
552 8th Avenue
San Francisco California 94118(US)

(74) Representative: Armitage, Ian Michael et al,
MEWBURN ELLIS & CO. 2/3 Cursitor Street
London EC4A 1BQ(GB)

## (54) Human prorelaxin, its preparation and use.

(57) Human prorelaxin is disclosed as a therapeutic agent, for example in effecting parturition and treating connective tissue disorders. also disclosed are means and methods for recombinant expression of human prorelaxin.

EP 0 260 149 A1

0260149

# HUMAN PRORELAXIN, ITS PREPARATION AND USE

This invention relates to human prorelaxin and compositions thereof and to the means and methods for its production to homogeneity in therapeutically significant quantities.

The present invention arises from the discovery that human prorelaxin lengthens the murine pubic symphysis *in vivo* and elevates cAMP levels in a newborn rhesus monkey uterine cell line. This discovery suggests that prorelaxin is active in remodelling the reproductive tract before parturition, thus facilitating the birth process.

In another aspect this invention is directed to the first successful use of genetic engineering, specifically recombinant DNA techniques, for expression of human prorelaxin. Yet another aspect of this invention is the purification of prorelaxin. Still another aspect of this invention is the therapeutic use of human prorelaxin.

Mature human relaxin is an ovarian peptide known to be responsible for remodelling the reproductive tract before parturition, thus facilitating the birth process. Hisaw, F.L., Proc. Soc. Exp. Biol. Med. 23, 661-663 (1926); Schwabe, C. *et al*., Biochem. Biophys. Res. Comm. 75, 503-570 (1977); James R. *et al*., Nature 267, 544-546 (1977). While predominantly a hormone of pregnancy, relaxin has also been detected in the non-pregnant female as well as in the male. Bryant-Greenwood, G.D., Endocrine Reviews 3, 62-90 (1982) and Weiss, G., Ann. Rev. Physiol. 46, 43-52 (1984). The hormone consists of two peptide chains, referred to as A and B, joined by disulfide bonds with an intra-chain disulfide loop in the A-chain in a manner analogous to that of insulin. Unless otherwise noted, "relaxin" shall mean the processed, two-chain molecule.

The application of recombinant DNA technology has led to the isolation and characterization of the genes coding for human relaxin. Hudson, P. *et al*., Nature 301, 628-631 (1984) and Hudson, P. *et al*., The EMBO Journal 3, 2333-2339 (1984). Analysis of the nucleotide sequence from cDNA and genomic clones reveals the structural organization of human relaxin to include a signal peptide of 25 residues, followed by a B-chain of about 32 or 33 amino acids, a C-peptide of about 105 amino acids, and an A-chain of 24 amino acids. The protein comprising the signal sequence, B-chain, C-peptide and A-chain is referred to as preprorelaxin. The protein comprising the B-chain, C-peptide and A-chain is referred to as prorelaxin. In the case of genomic DNA encoding human preprorelaxin an intron interrupts the coding region of the C-peptide. The physiological role of the C-peptide which is considerably longer than the C-peptide of insulin, and the nature of the enzymes responsible for removal of the C-peptide from the ends of the A- and B-chain are unresolved issues.

In humans, the primary source of relaxin is the corpus luteum. Weiss, G., "Human Relaxin: Source and Stimulus" in Bryant-Greenwood, G.D. *et al*., eds. Relaxin (Elsevier North Holland, NY (1981). In the case of human relaxin two separate gene sequences have been identified. (Weiss, Id.) Only one of these genes (H2) was found to be consistently expressed in the ovary during pregnancy while the other gene (H1) has been found to be expressed rarely and at a low level in the ovary and somewhat more frequently at other tissues. It has been suggested that the H1 gene may represent a pseudo-gene. The two human relaxin genes show considerable nucleotide and amino acid homology to each other, particularly in the B- and C-peptide. However, there are some notable regions of sequence divergence, particularly in the amino terminal region of both A- and B-chains. The fact that H2 relaxin is synthesized and expressed in the ovary suggests that this is the sequence which is involved in the physiology of pregnancy.

Prorelaxin is, as are most prohormones, considered a precursor to relaxin. Hudson, P. et al., Nature 301, 628-631 (1983) identified a genomic clone for human relaxin (H1) and deduced the amino acid sequence for human preprorelaxin. They suggested that following processing of the signal sequence, the C-peptide is cleaved at the B/C and C/A junctions. Hudson et al., supra at 630, indicate that the "biologically active hormone" will have a B-chain of 32 residues and an A-chain of 24 residues. The same group, Hudson P., et al., EMBO J., 3(10), 2333-2339 (1984) identified the second human relaxin gene, referred to as H2. Their experimental results support the notion that biological activity resides in the A and B chains of H2 relaxin. Id. at 2337. They conclude that "H2 relaxin is involved in the physiology of pregnancy..." Id. at 2338. Frieden, E.H. and Yeh, L., Proc. Soc. Exptl. Bio. Med. 154, 407-411 (1977) present evidence for the presence of a "pro-relaxin" in porcine relaxin concentrates. They express uncertainty as to whether the precursor itself possesses activity or the activity observed is due to conversion of prorelaxin to relaxin. Relaxin has long been known to act on the pubic symphysis and has been shown to have a remodeling effect using a murine pubic symphysis in vitro bioassay. Steinetz, B., et al. Endocrinology 67,102(1960). Relaxin is known to affect the dilatation of the cervix and render the uterus quiescent using rat uterine smooth muscle in an _in vitro_ assay. St. Louis, J. Can J. Physiol. Pharmacol. _59_, 507-512 (1981). It is thought that the effects of relaxin on the pregnant female are via an alteration in collagen in target tissues. This effect is thought to be mediated by certain cells such as fibroblasts, muscle cells and perhaps myofibroblasts all of which synthesize collagen. On a molecular level these affects may be mediated by cyclic adenosine 3',5' monophosphate (cAMP). Measurement of cAMP levels after a hormonal stimulus has been used as a marker for the biological affect of a peptide, particularly relaxin.

The exclusive action of cAMP is thought to be the activation of cAMP-dependent protein kinases. Kuo _et al_., PNAS (USA) _64_, 1349-1355 (1969) and Insel _et al_., Science _190_, 896-898 (1975). cAMP-dependent protein kinases have been described in the uterus and are present in both the myometrium, Korenman _et al_., Science _183_, 430-432 (1974), and the endometrium Sanborn _et al_., J. Biol. Chem. _248_, 3593-3600 (1973).

The ovarian peptide hormone relaxin has been shown to elevate cAMP in the mouse pubic symphysis, Braddon, S.A., Endocrinology _102_, 1292-1299 (1978); rat, Judson _et al_., J. Endocrinol. _87_, 153-159 (1980) and Sanborn _et al_., _supra_; and, rat myometrial cells, Hsu _et al_., Endocrinology _116_, 2029-2035 (1985).

All assay systems demonstrating the relationship of relaxin with cAMP production thus far reported have employed either uterine tissue sections or primary culture of uterine cells. Animal to animal variation and differences in the cell type mixture of primary cultured cells (i.e. connective tissue, medial vascular myometrium and inner circular and outer longitudinal myometrial cells) can significantly affect the assay results. Leroy _et al_., Acta Physiologica Hungarica _67_, 83-94 (1986). One aspect of the instant invention was to identify and develop a mammalian cell line which would respond to prorelaxin stimulation by elevation of cAMP levels.

Human prorelaxin has not been isolated from human tissue. This invention which provides the first successful expression of human prorelaxin using recombinant techniques enabled the discovery that human prorelaxin is biologically active. It has now been discovered that human prorelaxin positively effects the murine pubic symphysis ligament in vivo and elevates cAMP levels in newborn rhesus monkey uterus cell line (NRMU), thereby leading us to conclude that prorelaxin is biologically active in its own right. This result is new and unexpected in light of the proposed and demonstrated functions of most prohormones: stabilization of the three dimensional configuration of the peptide chain; assuring proper formation of the intramolecular bonds that are subsequently formed; facilitation of intracellular transport and packaging into vesicles; increased resistance to enzymatic degradation and excretion and consequent longer half-life when in the bloodstream; and properties similar to the hormone but with a lower potency. Martin, C.R., Endocrine Physiology (Oxford University Press, N.Y., 1985). The discovery that establishes prorelaxin as a hormone in its own right is to be contrasted to the traditional notions which relegate prohormones to mere precursors of biologically active hormones. As such, prorelaxin is believed to be biologically active in other physiological arenas besides the birth process e.g. wound healing; skin disease and connective tissue cancer.

Furthermore, the invention relates to the first successful application of recombinant DNA technology to produce human prorelaxin. In still another aspect the invention comprises pure human prorelaxin produced by recombinant DNA technology. Finally the human prorelaxin of this invention is useful in modulating the reproductive physiology of human beings and other mammals, including but not limited to maintenance of pregnancy, to effect parturition and enhancement of sperm motility as an aid in fertilization.

Brief Description of the Drawings

Figure 1. Method of obtaining a DNA segment encoding prorelaxin having the ATG position at the 5' translation initiation site of prorelaxin.

Figure 2. Construction of the expression vector pSV preprorelaxin 157.

Figure 3. Construction of intermediate expression vector pF8CIS.

Figure 4. Construction of a prorelaxin expression vector pCIHRX.

Figure 5. Construction of a prorelaxin expression vector pCISRX.

Figure 6. Enzyme Linked Immunosorbent Assay (ELISA) of prorelaxin purified from transformed CHO cells.

Figures 7a. and 7b. Coomassie blue and Western immunoblot gels showing isolation of a protein corresponding to prorelaxin having a molecular weight of about 17,000 daltons.

Figures 8a. and 8b. Spectrophotometric (absorbance at 280 nm) and ELISA results of further purification of prorelaxin following FPLC.

Figure 9. Gel electrophoresis of pooled fractions following FPLC of prorelaxin.

Figure 10. Results of murine pubic symphysis bioassay of human prorelaxin.

Figure 11. Nucleotide and amino acid sequences of human preprorelaxin.

## Detailed Description

**Definitions**

As used herein human prorelaxin denotes a functional protein capable of exhibiting a biological activity. Human prorelaxin biological activity is defined as any of 1) immunological cross-reactivity with at least one epitope of human prorelaxin or 2) the possession of at least one hormonal function in common with human relaxin. The hormonal functions of human relaxin and human prorelaxin as established by the discovery of this invention are: to effect numerous reproductive functions relating to pregnancy as well as other biological functions such as: preparation of the birth canal by, for example, effects on the pubic symphysis, pubic ligament and rearrangement of collagenous filaments thus effecting parturition; depressant effects on the myometrium; preparation of the endometrium for implantation; role in luteolysis; growth and differentiation of the mammary glands; enhancement of sperm motility; may augment the ability of sperm to penetrate the human cervix; connective tissue disease such as scleroderma, Peyronie's disease and fibrocytis; tendon disease, e.g. carpal tunnel syndrome; contractive deformities; back pain; and may improve skin elasticity, e.g. aging skin therapeutic. The human prorelaxin was produced by recombinant cell culture in active form(s) corresponding to human prorelaxin native to human plasma. The human prorelaxin protein produced herein is further defined by amino acid sequence and physical characteristics.

Two indicia of the functionality of human prorelaxin or variants thereof is evidenced by a positive effect in the murine pubic symphysis *in vitro* bioassay and/or an increase in cAMP levels in an *in vitro* assay using a uterine cell line.

Human prorelaxin is defined to be a molecule having the amino acid sequence shown in Figure 11 and includes amino acid sequence variants which maintain the functional characteristics discussed above. These variants may comprise one or more amino acid differences in the overall sequence or by deletions, substitutions and/or insertions of one or more amino acids in the overall sequence. A prorelaxin variant made by deletion of amino acids from the C chain includes a prorelaxin variant comprising the A and B chains of prorelaxin in a single chain and excludes the two chain form of mature human relaxin. Through the use of recombinant DNA technology human prorelaxin variants may be prepared by altering the underlying DNA. All such variations or alterations in the structure of human prorelaxin resulting in human prorelaxin variants are included within the scope of this invention so long as the functional activity of human prorelaxin is maintained. Those variants of human prorelaxin having the described functional activity can be readily identified using the in vitro cAMP assay described herein.

"Essentially pure" or "essentially free" when used to describe human prorelaxin produced by the method of the instant invention means substantially free of protein or other materials ordinarily associated with human prorelaxin when isolated from non-recombinant sources of plasma or tissue ordinarily greater than or equal to 95% of the total protein being prorelaxin by weight.

As used herein, "nucleotide sequence" refers to a nucleic acid comprising a series of nucleotides in a 5' to 3' phosphate diester linkage which may be either an RNA or a DNA sequence. If a DNA, the nucleotide sequence may be either single or double stranded. Similarly, "DNA sequence" refers to both single and double stranded embodiments.

"Control region" refers to specific sequences at the 5' and 3′ ends of eukaryotic genes which may be involved in the control of either transcription of translation. Virtually all eukaryotic genes have an AT-rich region located approximately 25 to 30 bases upstream from the site where transcription is initiated. Another sequence found 70 to 80 bases upstream from the start of transcription is a CXCAAT region where X may be any nucleotide. At the 3′ end of most eukaryotic genes is an AATAAA sequence which may be the signal for addition of the polyadenylation tail to the 3′ end of the transcribed mRNA.

"Promoter" refers to the nucleotide segment recognized by RNA polymerase molecules that start RNA synthesis. Promoters suitable for use with prokaryotic hosts include the β-lactamase and lactose promoter systems (Chang et al., 1978, "Nature", 281: 544), alkaline phosphatase, a tryptophan (trp) promoter system (Goeddel, 1980, "Nucleic Acids Res." 8: 4057 and EPO Appln. Publ. No. 36,776) and hybrid promoters such as the tac promoter (H. de Boer et al., 1983, "Proc. Natl. Acad. Sci. USA" 80: 21-25). However, other known bacterial promoters are suitable. Their nucleotide sequences have been published, thereby enabling a skilled worker operably to ligate them to DNA encoding prorelaxin (Siebenlist et al., 1980, "Cell" 20: 269) using linkers or adaptors to supply any required restriction sites. Promoters for use in bacterial systems also will contain a Shine-Dalgarno (S.D.) sequence operably linked to the DNA encoding prorelaxin.

Suitable promoting sequences for use with yeast hosts include the promoters for 3-phosphoglycerate kinase (Hitzeman et al., 1980, "J. Biol. Chem." 255: 2073) or other glycolytic enzymes (Hess et al., 1968, "J. Adv. Enzyme Reg." 7: 149; and Holland, 1978, "Biochemistry", 17: 4900), such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase.

Other yeast promoters, which are inducible promoters having the additional advantage of transcription controlled by growth conditions, are the promoter regions for alcohol dehydrogenase 2, isocytochrome C, acid phosphatase, degradative enzymes associated with nitrogen metabolism, metallothionein, glyceraldehyde-3-phosphate dehydrogenase, and enzymes responsible for maltose and galactose utilization. Suitable vectors and promoters for use in yeast expression are further described in R. Hitzeman et al., EP 73,657A. Yeast enhancers also are advantageously used with yeast promoters.

The preferred promoters controlling transcription of DNA encoding prorelaxin from vectors in mammalian host cells may be obtained from the genomes of viruses such as: polyoma, Simian Virus 40 (SV40), adenovirus, retroviruses, hepatitis-B virus and most preferably cytomegalovirus, or from heterologous mammalian promoters, e.g. beta actin promoter. The early and late promoters of the SV40 virus are conveniently obtained as an SV40 restriction fragment which also contains the SV40 viral origin of replication. Fiers et al., 1978, "Nature", 273:113. The immediate early promoter of the human cytomegalovirus is conveniently obtained as a HindIII E restriction fragment. Greenaway, P.J. et al., Gene 18, 355-360 (1982). Of course, promoters from the host cell or related species also are useful herein.

"Enhancer" refers to cis-acting elements of DNA, usually about from 10-300 bp, that act on a promoter to increase its transcription. Transcription of a DNA encoding a desired factor VIII variant by higher eukaryotes is increased by inserting an enhancer sequence into the vector. Enhancers are relatively orientation and position independent having been found 5' (Laimins, L. et al., PNAS 78, 993 [1981]) and 3' (Lusky, M.L., et al., Mol. Cell Bio. 3, 1108 [1983]) to the transcription unit, within an intron (Banerji, J.L. et al., Cell 33, 729 [1983]) as well as within the coding sequence itself (Osborne, T.F., et al., Mol. Cell Bio. 4, 1293 [1984]). Many enhancer sequences are now known from mammalian genes (globin, elastase, albumin, α-fetoprotein and insulin). Typically, however, one will use an enhancer from a eukaryotic cell virus. Examples include the SV40 enhancer on the late side of the replication origin (bp 100-270), the cytomegalovirus early promoter enhancer, the polyoma enhancer on the late side of the replication origin, and adenovirus enhancers.

Preferred expression vectors used in eukaryotic host cells (yeast, fungi, insect, plant, animal, human or nucleated cells from other multicellular organisms) will also contain sequences necessary for the termination of transcription which may affect mRNA expression. These regions are transcribed as polyadenylated segments in the untranslated portion of the mRNA encoding the desired factor VIII variant. The 3' untranslated regions also include transcription termination sites.

Expression vectors may contain a selection gene, also termed a selectable marker. A selection gene encodes a protein, sometimes referred to as a secondary protein, necessary for the survival or growth of a host cell transformed with the vector. Examples of suitable selectable markers for mammalian cells are dihydrofolate reductase (DHFR), thymidine kinase or neomycin. When such selectable markers are successfully transferred into a mammalian host cell, the transformed mammalian host cell can survive if placed under selective pressure. There are two widely used distinct categories of selective regimes. The first category is based on a cell's metabolism and the use of a mutant cell line which lacks the ability to grow independent of a supplemented media. Two examples are: CHO $DHFR^-$ cells and mouse $LTK^-$ cells. These cells lack the ability to grow without the addition of such nutrients as thymidine or hypoxanthine. Because these cells lack certain genes necessary for a complete nucleotide synthesis pathway, they cannot survive unless the missing nucleotides are provided in a supplemented media. An alternative to supplementing the media is to introduce an intact DHFR or TK gene into cells lacking the respective genes, thus altering their growth requirements. Individual cells which were not transformed with the DHFR or TK gene will not be capable of survival in non supplemented media. Therefore, direct selection of those cells requires cell growth in the absence of supplemental nutrients.

The second category is dominant selection which refers to a selection scheme used in any cell type and does not require the use of a mutant cell line. These schemes typically use a drug to arrest growth of a host cell. Those cells which have a novel gene would express a protein conveying drug resistance and would survive the selection. Examples of such dominant selection use the drugs neomycin, Southern P. and Berg, P., J. Molec. Appl. Genet. 1, 327 (1982), mycophenolic acid, Mulligan, R.C. and Berg, P. Science 209, 1422 (1980) or hygromycin, Sugden, B. *et al.*, Mol. Cell. Biol. 5:410-413 (1985). The three examples given above employ bacterial genes under eukaryotic control to convey resistance to the appropriate drug neomycin (G418 or geneticin), xgpt (mycophenolic acid) or hygromycin, respectively. The amount of each drug necessary to arrest growth may vary depending on the cell type. Therefore, drug levels are usually titrated to determine the lethal dose required to allow selective survival of cells which have retained the transfected gene conveying resistance. In the foregoing experiments the selective agent of choice is most often G418 geneticin unless specifically referring to CHO $DHFR^-$ cells. In this case the direct selection for DHFR production was used.

"Amplification" refers to indreases in the number of tandem copies of a region within a cell's chromosomal DNA. Amplification is achieved using a selection agent e.g. methotrexate (MTX) which inactivates DHFR. Amplification of the DHFR gene results in greater amounts of DHFR being produced in the face of greater amounts of MTX. Amplification pressure is applied notwithstanding the presence of endogenous DHFR, by providing ever greater concentrations of MTX in the media. Amplification of a desired gene can be achieved by cotransfecting a mammalian host cell with a plasmid having a DNA encoding a desired protein and the DHFR or amplifiable gene so that cointegration can occur. One ensures that the cell requires more DHFR, which requirement is met by replication of the amplifiable gene, by selecting only for cells that can grow in successive rounds of ever-greater MTX concentration. So long as the gene encoding a desired heterologous protein has cointegrated with the selection gene replication of this gene gives rise to replication of the gene encoding the desired protein. The result is that increased copies of the gene, i.e. an amplified gene, encoding the desired heterologous protein express more of the desired heterologous protein.

Suitable host cells for cloning or expressing the vectors herein are prokaryotes, yeast or higher eukaryotic cells. Prokaryotes include gram negative or gram positive organisms, for example E. coli 294 (ATCC 31,446) although other gram negative or gram positive prokaryotes such as E. coli B, E. coli X1776 (ATCC 31,537), E. coli W3110 (ATCC 27,325), pseudomonas species, or Serratia Marcesans are suitable.

In addition to prokaryotes, eukaryotic microbes such as filamentous fungi or yeast are suitable hosts for prorelaxin variant vectors. Saccharomyces cerevisiae, or common baker's yeast, is the most commonly used among lower eukaryotic host microorganisms. However, a number of other genera, species and strains are commonly available and useful herein.

Suitable host cells for expressing prorelaxin in higher eukaryotes include: monkey kidney CVI line transformed by SV40 (COS-7, ATCC CRL 1651); human embryonic kidney line (293, Graham, F.L. et al. J. Gen Virol. 36, 59 [1977]); baby hamster kidney cells (BHK, ATCC CCL 10); chinese hamster ovary-cells-DHFR (described by Urlaub and Chasin, PNAS (USA) 77, 4216, [1980]); mouse sertoli cells (TM4, Mather, J.P., Biol. Reprod. 23, 243-251 [1980]); monkey kidney cells (CVI ATCC CCL 70); african green monkey kidney cells (VERO-76, ATCC CRL-1587); human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51); rat hepatoma cells (HTC, M1, 54, Baumann, M. et al., J. Cell Biol. 85, 1-8 [1980]) and, TRI cells (Mather, J.P. et al., Annals N.Y. Acad. Sci. 383, 44-68 [1982]).

"Transformation" means introducing DNA into an organism so that the DNA is replicable, either as an extrachromosomal element or by chromosomal integration. Unless otherwise provided, the method used herein for transformation of the host cells is the method of Graham, F. and van der Eb, A., Virology 52, 456-457 (1973).

Host cells may be transformed with the expression vectors of the instant invention and cultured in conventional nutrient media modified as is appropriate for inducing promoters, selecting transformants or amplifying genes. The culture conditions, such as temperature, pH and the like, are those previously used with the host cell selected for expression, and will be apparent to the ordinarily artisan.

"Transfection" refers to the taking up of an expression vector by a host cell in a detectable manner whether or not any coding sequences are in fact expressed. Numerous methods of transfection are known to the ordinarily skilled artisan, for example, $CaPO_4$ and electroporation. Successful transfection is generally recognized when any indication of the operation of this vector occurs within the host cell. However, in the context of the present invention successful transfection refers to stable continuous expression of a desired heterologous protein by a host culture over numerous generations.

An assay based on immunoperoxidase staining (Gorman, C.M. et al., Cell 42, 519-522 [1985]) of a transfected cell was developed to assess quickly whether prorelaxin had been expressed. Monoclonal antibodies specific for the prorelaxin were screened for use in this assay. Host cells containing the vector were stained and compared to parental cell line for screening cells which produce a specific protein. A monoclonal antibody was selected which gave the strongest signal with the least amount of background. Cells (Cos, 293, CHO, BHK, TM4) were transfected using the $CaPO_4$ technique. (Graham and van der Eb modified by Gorman, C.M. et al., Science 221, 551-553 [1983]). We used ten micrograms per milliliter of precipitate of the specific protein vector to be tested. The precipitates were left on the cells for 3-4 hours. Cells were then glycerol shocked for an average of one minute. Thirty-six hours after transfection cells were fixed with acetone-methanol (50:50) and washed with phosphate buffer saline (PBS). Staining was performed using either a monoclonal antibody supernatant undiluted or purified antibody diluted 1:3000 in PBS containing 10% fetal calf serum. This first antibody remained on the cells for 2 hours. Plates were placed on a slow shaker during this time. Cells were washed 5 times over a ten minute period. The second antibody used was rabbit anti-mouse IgG (Dakopatts). This was diluted in PBS + fetal calf serum at a dilution of 1:150. A two hour incubation was followed by another series of washes. To develop the peroxidase reagent ortho-diansidine was used as a substrate. A ethanol saturated solution of ortho-diansidine was diluted 1:100 in PBS with 1:10,000 dilution of hydrogen peroxide. This substrate was left on the cells for 2 hrs at room temperature or overnight at 4°C.

In order to simplify the following examples certain frequently occurring methods and/or terms will be described.

"Plasmids" are designated by a lower case p preceded and/or followed by capital letters and/or numbers. The starting plasmids herein are either commercially available, publicly available on an unrestricted basis, or can be constructed from available plasmids in accord with published procedures. In addition, equivalent plasmids to those described are known in the art and will be apparent to the ordinarily skilled artisan.

"Digestion" of DNA refers to catalytic cleavage of the DNA with an enzyme that acts only at certain sequences in the DNA. Such enzymes are called restriction enzymes, and the specific cleavage site for which each is specific is called a restriction site. The various restriction enzymes used herein are commercially available and their reaction conditions, cofactors and other requirements were used as would be known to the ordinarily skilled artisan. Restriction enzymes are designated by abbreviations composed of a capital letter followed by other letters representing the microorganism for which each restriction enzyme originally was obtained and then a number designating the particular enzyme. In general, about 1 $\mu g$ of plasmid or DNA fragment is used with about 2 units of enzyme in about 20 $\mu l$ of buffer solution. Appropriate buffers and substrate amounts for particular restriction enzymes are specified by the manufacturer. Incubation times of about 1 hour at 37°C are ordinarily used, but may vary in accordance with the supplier's instructions. After digestion the reaction was run directly on a gel to isolate the desired fragment.

"Dephosphorylation" refers to the removal of the terminal 5' phosphates by treatment with bacterial alkaline phosphatase (BAP). This procedure prevents the two restriction cleaved ends of a DNA fragment from "circularizing" or forming a closed loop that would impede insertion of another DNA fragment at the restriction site. Procedures and reagents for dephosphorylation are conventional. Maniatis, T. et al., 1982, Molecular Cloning pp. 133-134. Reactions using BAP are carried out in 50mM Tris at 68°C to suppress the activity of any exonucleases which may be present in the enzyme preparations. Reactions were run for 1 hour. Following the reaction the DNA fragment is gel purified. Removal of the 5' phosphate groups was carried out to prevent reclosure of the vector to allow the insertion of additional DNA.

"Oligonucleotides" refers to short length single or double stranded polydeoxynucleotides which are chemically synthesized by known methods and then purified on polyacrylamide gels.

"Ligation" refers to the process of forming phosphodiester bonds between two double stranded nucleic acid fragments (Maniatis, T. *et al.*, *Id.*, p. 146). Unless otherwise provided, ligation may be accomplished using known buffers and conditions with 10 units of T4 DNA ligase ("ligase") per 0.5 $\mu$g of approximately equimolar amounts of the DNA fragments to be ligated.

"Filling" or "blunting" refers to the procedures by which the single stranded end in the cohesive terminus of a restriction enzyme-cleaved nucleic acid is converted to a double strand. This eliminates the cohesive terminus and forms a blunt end. This process is a versatile tool for converting a restriction cut end that may be cohesive with the ends created by only one or a few other restriction enzymes into a terminus compatible with any blunt-cutting restriction endonuclease or other filled cohesive terminus. Typically, blunting is accomplished by incubating 2-15$\mu$g of the target DNA in 10mM $MgCl_2$, 1mM dithiothreitol, 50mM NaCl, 10mM Tris (pH 7.5) buffer at about 37°C in the presence of 8 units of the Klenow fragment of DNA polymerase I and 250 $\mu$M of each of the four deoxynucleoside triphosphates. The incubation generally is terminated after 30 min. phenol and chloroform extraction and ethanol precipitation.

The following examples merely illustrate the best mode now known for practicing the invention, but should not be construed to limit the invention. All literature citations herein are expressly incorporated by reference.

## Example 1

## Prorelaxin Expression Vector

The preprorelaxin DNA could be excised from the cloning vector as described in Hudson, P. *et al.*, The EMBO Journal 3, 2333-2339, by using a complete HpaII digest followed by a HinfI partial digest. A single stranded DNA primer of the sequence 5'-ATGCCTCGCCTGTTT-3' (ATG primer) was synthesized, mixed with the preprorelaxin DNA segment, the mixture boiled in order to separate the strands of the preprorelaxin segment, and cooled to allow the ATG-primer to anneal with the anti-sense DNA strand of preprorelaxin. See Figure 1.

The Klenow fragment of DNA polymerase I was then used to extend the ATG-primer in the 3' direction close to the end of the preprorelaxin DNA segment. In addition, the 3'->5' exonucleolytic activity of the Klenow fragment degraded the 3' end at the preprorelaxin anti-sense strand exactly up to the nucleotide which is base paired to the 5' terminal 'A' of the ATG primer, thus making this terminus blunt ended. This new double stranded preprorelaxin segment was digested at an AvaII site in the 3' untranslated region of the preprorelaxin segment, and the AvaII terminus filled in with the Klenow fragment enzyme to make this end blunt ended.

The fragment was then blunt-end ligated into the plasmid trp207-1*tetxap (Gray, G.L. *et al.*, Gene 39: 247-254[1985]) which had been cleaved with EcoRI and its termini filled in with the Klenow fragment enzyme. See Figure 2. The plasmid described by Gray *et al.*, supra had an AvaI-PvuII segment deleted and an EcoRI site at the 5' side of the trp promoter removed. After selecting plasmids containing the preprorelaxin segment in the correct orientation, the preprorelaxin trp207-1*tetxap plasmid was digested with ClaI and the termini filled in with Klenow. The linearized plasmid was further digested with XbaI and the preprorelaxin segment isolated by gel electrophoresis.

The eucaryotic expression vector p342E (Crowley, C.W. et al., Mol. Cell. Biol. 3: 44-55 [1983]) was digested with BamHI, the termini filled in with Klenow, the vector further digested with XbaI, and the vector fragment isolated by gel electrophoresis. The p342E plasmid was modified by addition of a DNA coding DHFR as described by Patzer, E.J. et al., J. Virol. 51, 346-353 (1984). The plasmid described in Crowley et al., supra had an XbaI site added following the SV40 early promoter using a synthetic polylinker (commercially available from New England Biolabs). This plasmid was labeled pSVCV202 as shown in Figure 2. This vector fragment was then ligated with the XbaI-ClaI preprorelaxin fragment, generating the eucaryotic expression plasmid pSV preprorelaxin 157 (also referred to as "pSVERX").

The expression plasmid containing the gene for H2 preprorelaxin, an SV40 early promoter and the gene encoding the selectable marker dihydrofolate reductase (pSVERX) was transfected into CHO-DX-B11 Cells ($DHFR^-$) by electroporation (Potter et al., PNAS 81. 7161. [1984]).

One confluent 100mm culture dish of CHO-DX-B11 cells was trypsinized with 2ml of phosphate buffered saline (PBS; 0.15M NaCl-0.015M NaPO4, pH7.2) and 0.5mg/ml bovine trypsin. The cells were added to 10ml F12 culture medium (commercially available from Gibco) containing 10% fetal bovine serum, washed twice with PBS, and suspended in 1.5ml PBS. The cell suspension was placed on ice and 30mg of the expression plasmid, pSVERX, was added.

One-third of the DNA-cell mixture was transferred to a 1 ml plastic cuvette fitted with an aluminum foil electrode and given an electric pulse of 2000 volts using an ISCO 494 power supply. The transfection mixture was transferred to two 100mm culture dishes each containing 14ml of non-selective growth medium (F-12-10% fetal bovine serum [FBS], glycine-hypoxanthine-thymidine [GHT]). After 2 days the medium was changed to selective media (F-12-10% dialysed FBS) in order to select those cells expressing the DHFR gene. Surviving cell efficiencies were on the order of about 2,000 colonies per plate.

**Example 2**

**Construction of Human Prorelaxin Vector**

The vector pCIHRX contains the cytomegalovirus (CMV) enhancer and promoter, the cytomegalovirus splice donor site, the Ig variable region splice acceptor site, the cDNA encoding H2 preprorelaxin and the hepatitis surface antigen polyadenylation site. Figures 3, 4 and 5 show the steps for construction of the prorelaxin expression vector used to establish production cell lines. The construction is detailed below beginning with construction of the starting plasmid pF8CIS shown in Figure 3.

1) The ampicillin resistance marker and replication origin of the final vector was derived from the starting plasmid pUC13pML a variant of the plasmid pML (Lusky, M. and Botchen, M., Nature 293, 79 [1981]). pUC13pML was constructed by transferring the polylinker of pUC13 (Veira, J. and Messing, J., Gene 19:259 [1982]) to the EcoRI and HindIII sites of pML. A second starting plasmid pUC8CMV was the source of the CMV enhancer, promoter and splice donor sequence. pUC8CMV was constructed by inserting nucleotides for the CMV enhancer, promoter and splice donor sequence into the blunted PstI and SphI sites of pUC8. Veira, J. and Messing, J. supra. Synthetic BamHI-HindIII linkers (commercially available from New England Biolabs) were ligated to the cohesive BamHI end creating a HindIII site. Following this ligation a HindIII-HincII digest was performed. This digest yielded a fragment of approximately 800bp which contained the CMV enhancer, promoter and splice donor site. Following gel isolation this 800bp fragment was ligated to a 2900bp piece of pUC13pML. The fragment required for the construction of pF8CIS was obtained by digestion of the above intermediate plasmid with SalI and HindIII. This 3123bp piece contained the resistance marker for ampicillin, the origin of replication from pUC13pML and the control sequences for the CMV including the enhancer, promoter and splice donor site.

2) The Ig variable region intron and splice acceptor sequence was constructed using a synthetic oligomer as shown in the central portion of Figure 3. A 99 mer and a 30 mer were chemically synthesized having the following sequence for the IgG intron and splice acceptor site (Bothwell *et al.*, 1981):

1 5'AGTAGCAAGCTTGACGTGTGGCAGGCTTGA...
31 GATCTGGCCATACACTTGAGTGACAATGA...
60 CATCCACTTTGCCTTTCTCTCCACAGGT...
88 GTCCACTCCCAG3'

1 3'CAGGTGAGGGTGCAGCTTGACGTCGTCGGA5'

DNA polymerase I (Klenow fragment) filled in the synthetic piece and created a double stranded fragment. Wartell, R.M. and W.S. Reznikoff, Gene 9, 307 (1980). This was followed by a double digest of PstI and HindIII. This synthetic linker was cloned into pUC13 (Veira, J. and Messing, J., Gene 19, 259 [1982]) at the PstI and HindIII sites. The clone containing the synthetic oligonucleotide, labelled pUCIg.10, was digested with PstI. A ClaI site was added to this fragment by use of a PstI-ClaI linker. Following digestion with HindIII a 118bp piece containing part of the Ig intron and the Ig variable region splice acceptor was gel isolated.

3) The third part of the construction scheme replaced the hepatitis surface antigen 3' end contained in pSVE.8cID (European Patent Publication No. 160,457) with the polyadenylation site of the early region of SV40. A vector, pUC.SV40 containing the SV40 sequences was inserted into pUC8 at the BamHI site described in Viera, J. and Messing, J., supra. pUC.SV40 was then digested with EcoRI and HpaI. A 143bp fragment was gel isolated from this digest. Two additional fragments were gel isolated following digestion of pSVE.8C1D. European Patent Publication No. 160,457. The 4.8 kb fragment generated by EcoRI-and ClaI digest contains the SV40-DHFR transcription unit, the origin of replication of pML and the ampicillin resistance marker. The 7.5 kb fragment produced following digestion with ClaI and HpaI contains the cDNA for factor VIII. A three-part ligation yields pSVE.8C24D. This intermediate plasmid was digested by ClaI and SalI to give a 9611bp fragment containing the cDNA for factor VIII with an SV40 polyadenylation site followed by the SV40 DHFR transcription unit.

The final three part ligation to yield pF8CIS used: a) the 3123bp SalI HindIII fragment containing origin of replication, the ampicillin resistance marker and the CMV enhancer, promoter and splice donor; b) the 118bp HindIII-ClaI fragment containing the Ig intron and splice acceptor; and, c) the 9611bp ClaI-SalI fragment containing the cDNA for factor VIII, SV40 polyadenylation site and the SV40 DHFR transcription unit.

1. pCIHRX

The vector pCIHRX contained the cytomegalovirus enhancer and promoter, the cytomegalovirus splice donor site, the Ig variable region splice acceptor site, the cDNA encoding H2 preprorelaxin and the hepatitis surface antigen polyadenylation and transcription termination sites. Figure 4 shows the steps for construction of this vector. 677bp of the preprorelaxin cDNA followed the aforementioned 5' processing signals. While the 5' control signals were identical to pF8CIS the polyadenylation region and transcription termination sequence signals were from the hepatitis surface antigen gene rather than SV40.

An intermediate plasmid pClARX was first constructed. The plasmid pSVERX was cut with HindIII to isolate a 1700bp fragment containing the preprorelaxin cDNA followed by the hepatitis B surface antigen (HBsAg) 3' polyadenylation site. A KpnI site was 3' to the HBsAg polyadenylation site and 5' to the start of the SV40 early promoter which in this vector was used to drive expression of the DHFR cDNA.

This HindIII fragment was inserted into pML linearized at the HindIII site. Reclosures were minimized by treatment with bacterial alkaline phosphatase (BAP). Ampicillin resistant colonies were screened to isolate clones which had inserted the preprorelaxin gene so that the 5' end of the gene was next to the ClaI site of pML.

An alternative method for the construction of an expression vector for providing a source of the preprorelaxin cDNA uses the λGT10 cDNA library of Hudson et al., supra. Clone "a" contains a 1900bp insert which includes the complete cDNA for preprorelaxin. A complete HpaII digest followed by a HinfI partial digest will yield a fragment of approximately 650bp which encompasses from -100 to +660 of the preprorelaxin cDNA. The 5' and 3' flanking regions are modified by Bal31 and blunted with S1 nuclease to remove noncoding sequences and provide blunt ends. The mammalian expression vectors of the instant invention include 9bp upstream of the translation initiation codon and 88bp downstream of the TGA stop codon of the preprorelaxin cDNA. This modified cDNA can be used to construct pCLARX by modifying the 5' end of the cDNA with HindIII linkers (available from New England Biolabs). A final 3 part ligation will yield pCLARX: 1) cDNA preprorelaxin (HindIII 5' - blunt 3' end) 640bp; 2) a 990 bp including the (HBsAg) 3' polyadenylation site fragment of pETPFR (U.K. patent 2,119,804), cut with SacII - blunted with T4 polymerase and cut with HindIII, and 3) a HindIII linear of pML treated with BAP to prevent reclosure.

The intermediate plasmid pClARX was cut with ClaI and KpnI to isolate a 1360bp fragment containing the preprorelaxin gene followed by the hepatitis surface antigen 3' polyadenylation sequences. This fragment was ligated to the 5143bp fragment created by cutting pF8CIS dam$^-$ with ClaI and KpnI.

2. pCISRX

Because the choice of polyadenylation sequences is known to influence 5' processing of messenger RNA (Wilson & Nevins, supra), the 3' hepatitis polyadenylation sequence in pCIHRX was replaced with the pSV40 polyadenylation sequence. This construction was designated pCISRX and is shown in figure 5. The two starting vectors for this construction are pCIHRX and pF8CIS. The latter vector has the same 5' controls as pCIHRX but includes the cDNA for factor VIII and the SV40 polyadenylation site. SacII was used to cleave 3' of the cDNA. The resultant 3' overhang was blunted by T4 polymerase. pCIHRX was then cut with BamHI. This site separates the chimeric intron cleaving between the CMV intronic sequences and the Ig variable region intron. An 861bp fragment was gel isolated from the BamHI treatment. The SV40 polyadenylation site, DHFR, transcription unit, bacterial origin of replication and amp$^r$ gene, as well as the CMV enhancer and promoter and splice donor were isolated from pF8CIS. These elements were isolated in two fragments, as a 2525bp Sal-BamHI fragment and a HpaI-Sal 3113 bp fragment. A three part ligation of the BamHI-SacII (blunted) fragment with the HpaI-Sal fragment and Sal to BamHI fragment yields pCISRX.

## Example 3

## Expression Results

Prorelaxin expression was assayed using two methods. The first used an enzyme linked immunosorbent assay (ELISA) and the second immunoperoxidase staining. Each method is described below.

a) ELISA

Prorelaxin was monitored using antibodies raised in rabbits against synthetic relaxin ($Lys^3$, $Ala^{24}$ analogue; Ser N-terminal B-32) using the Enzyme Linked Immunoserbant Assay (ELISA) method (Engvall, E. and Perlmann, P., Immunochemistry 8, 871-874 (1971); Voller, A., et al. Bulletin of World Health 53, 55-65 (1976). The relaxin analogue was injected with complete Freund's adjuvant; booster immunizations were made with incomplete Freund's. Antibody was recovered from serum by precipitation with ammonium sulphate to 33% saturation, the precipitation repeated, and relaxin specific antibody purified by affinity chromatography with the same synthetic analogue relaxin used for immunization coupled to cyanogen bromide activated Sepharose 4B (Pharmacia). The relaxin specific antibody was eluted with 0.1M glycine pH 2.4 and was used both as the coat antibody and the conjugate antibody (conjugation method described in Immunoassays in the Clinical Laboratory, Method II, 81-87, Nakene, P. ed. [1979]).

Prorelaxin was monitored using antisera raised in rabbits against synthetic B-chain analogue ($Lys^3$, $Ala^{24}$; Ser N-terminal, B-32) using immunoblots and immunoprecipitation. The B-chain analogue was injected as a co-precipitate with alum, and serum collected after two boost injections.

The ELISA assayed one transfected culture plate directly for prorelaxin expression. The ELISA method was that of Engvall and Perlmann, supra. The plate was cultured to confluency, the media removed (three days accumulation, 14 ml.), centrifuged at 2,000 rpm for 5 minutes to remove debris, and duplicate samples were assayed in an ELISA designed to detect prorelaxin. The range of detection in the assay is 0.78-25 ng/ml. An identical control CHO culture absent the pSVERX plasmid was also assayed. The medium from the CHO- pSVERX culture measured 1.8 ng/ml of prorelaxin, while no relaxin was detected in the medium from control cultures.

A second culture plate was used to select colonies for amplification of the pSVERX plasmid by virtue of its DHFR gene. Increasing levels of methotrexate were used to put selective pressure on the pSVERX plasmid. Colonies were picked at ten levels of methotrexate in the range 10-200,000 ng/ml methotrexate. The colonies producing the highest levels of prorelaxin, as judged by the ELISA assay, were chosen at each stage for further growth and selection. A final clone grown in 200,000 ng/ml methotrexate was chosen after eight months of amplification. This clone produced about 100ng/ml prorelaxin, based on the ELISA assay described above, in a confluent culture over a three day period. Cell samples from this clone were frozen in liquid nitrogen.

An immunoprecipitation was performed on culture supernatants from the CHO cell line carrying the amplified preprorelaxin gene. Cultures of this cell line and of a control CHO cell line not carrying the human preprorelaxin gene were labelled with $^{35}S$-methionine (greater than 800 curies/mmole; Amersham). After adding 1/10th volume human serum, aliquots of the labelled supernatants were treated with 0.2 mg/ml Protein A-Sepharose (Pharmacia) and centrifuged at 10,000 x gravity for 2 minutes to remove non-specifically adsorbed proteins. To this supernatant was added 10 $\mu$l/ml of rabbit anti-sera raised against relaxin B-chain analogue, followed by 0.2 mg/ml Protein A-Sepharose. The immunoprecipitate was recovered by centrifugation at 10,000 x gravity for 2 minutes, washed 3 times with PBS-0.1 mg/ml BSA, suspended in BRL gel buffer and subjected to polyacrylamide gel electrophoresis in the BRL gel system. Bethesda Research Laboratories (Marciani, D. technical bulletin) and Shapiro, A.L. et al., Biochim. Biophys. Res. Comm. 28: 815-820 (1967). An autoradiogram of the dried gel (prepared with Enhance; New England Nuclear) is shown in figure 6. A band of approximately 17,000 daltons is present only from the cell line carrying the human preprorelaxin gene. This molecular weight is consistent with that of full length prorelaxin.

b) Immunoperoxidase

Prorelaxin expression was assayed based on immunoperoxidase staining of transfected cells. Gorman et al. Cell 42, 519-526 (1985). This assay was also used to test vectors for the expression of prorelaxin. Several specific monoclonal antibodies for prorelaxin were screened for use in this assay. One mouse monoclonal antibody IH6 (RX-1) was found to give the strongest signal with the lease amount of background. Transfections were performed and transient expression of prorelaxin was assessed. Cells (Cos, 293, CHO, BHK, TM4) were transfected using the $CaPO_4$ technique. Ten micrograms per milliliter of prorelaxin precipitate was tested. The precipitates were left on the cells for 3-4 hours. Cells were then glycerol shocked for an average of 1 minute. Thirty-six hours after transfection cells were fixed with acetone-methanol (50:50) and washed with phosphate buffered saline (PBS). Cells were stained using IH6 supernatant undiluted or purified IH6 antibody diluted 1:3000 in PBS containing 10% fetal calf serum. This first antibody remained on the cells for 2 hours. Plates were placed on a slow shaker during this time. Cells were washed 5 times over a ten minute period. A second antibody of rabbit anti-mouse IgG (Dakopatts) was diluted in PBS + fetal calf serum at a dilution of 1:150. A two hour incubation was followed by another series of washes. Ortho-diansidine (Sigma) was used as a substrate for developing the peroxidase reagent. An ethanol saturated solution of ortho-diansidine was diluted 1:100 in PBS with 1:10,000 dilution of hydrogen peroxide. This substrate was left on the cells for 2 hours at room temperature or overnight at 4°C. This method, used thirty-six hours after transfection, provided a screen for assessing the ability of a wide variety of prorelaxin vectors to direct transient prorelaxin expression.

The two vectors, pCIHRX and pCISRX, were tested for prorelaxin expression and compared to pSVERX. pCIHRX and pCISRX vectors differed in the polyadenylation sequence. pCIHRX contained the hepatitis surface antigen polyadenylation sequence while pCISRX contained the SV40 early region polyadenylation sequence.

293, TM4 and CHO cells were transfected with 10 μg total DNA which included 1 μg pRSVneo, 5 μg salmon sperm carrier and 4 μg of plasmids pSVERX, pCIHRX and pCISRX. Cells were glycerol shocked as described above. Thirty-six hours following transfection cells were fixed and stained with IH6 to identify transformed cells making prorelaxin. Positive staining cells were seen in 293 and TM4 cells transfected with pCIHRX and pCISRX. Duplicate plates of CHO, 293 and TM4 cells were split and subjected to the staining protocol described above to screen for prorelaxin production cells.

Expression results are shown in the tables below indicating that the vectors pCIHRX and pCISRX produced significantly higher levels of prorelaxin than the plasmid, pSVERX. In the case of stable expression the media assayed for prorelaxin using the ELISA assay described above, was from the general population of cells.

Transient Expression Prorelaxin

| Cell Type | Plasmid | Amount of Protein (ng/ml) |
| --- | --- | --- |
| CHO | pSVERX | 0.4 |
| | pCIHRX | 0.9 |
| | pCISRX | 3 |
| TM4 | pSVERX | 0.4 |
| | pCIHRX | 2 |
| | pCISRX | 10 |
| 293 | pSVERX | 0.4 |
| | pCIHRX | 3 |
| | pCISRX | 12 |

Stable Expression Prorelaxin

| Cell Type | Plasmid | Amount of Protein (ng/ml) |
| --- | --- | --- |
| CHO | pSVERX | 0.6 |
| | pCIHRX | 0.8 |
| | pCISRX | 3.9 |
| 293 | pSVERX | 0.41 |
| | pCIHRX | 3.0 |
| | pCISRX | 22.0 |

Example 4

Purification of Human Prorelaxin

The transformed CHO cell line producing prorelaxin (designated CHO.200.12) was thawed from liquid nitrogen and grown in culture dishes or flasks containing a supplemented culture medium. Any supplemented culture medium known to the ordinarily skilled artisan could be used. An example of such a medium is Earles minimal essential medium which could be supplemented with sodium bicarbonate, 10% dialyzed bovine serum and 10mm methotrexate.

These cultures were passaged (using trypsin) to either plastic roller bottles (850 $cm^2$) or to suspension culture vessels containing microcarrier beads as a surface for cell attachment. Up to 1000 liters and more of culture containing approximately 2mg/liter of prorelaxin has been obtained using these suspension culture methods.

The cell harvest media contained certain supplements such as insulin, transferrin, lipids, trace metals, guanine, hypoxanthine, and thymidine.

Prorelaxin was purified from 10-20 liters of culture supernatant (2mg/liter prorelaxin [based on mass]; 20mg-40mg total preprorelaxin). Supernatants were concentrated (reduced in volume), using a molecular sieving filter (Amicon, cm2) in a Pelicon pressure device. The volume was reduced by about 50-fold retaining greater than 80% of the prorelaxin in the concentrated fluid. After concentration, the salts were exchanged by repeated flushing of the concentration chamber with a buffer of choice.

The culture concentrate was fractionated by liquid chromatography. An ion exchange resin (such as PBE 118, Pharmacia) was packed into a glass column (1.4 cm x 50 cm) and equilibrated with a basic (high pH) buffer such as triethylamine (TEA). 25 mM TEA buffers from pH 10 to pH 11 have been used. The column was positively charged and at this pH (above the pI of prorelaxin), prorelaxin was negatively charged. Therefore, when the culture concentrate was passed through this column the majority of the prorelaxin binds to the resin by ionic interaction. After this material was passed through the column the column was rinsed with equilibration buffer.

Prorelaxin was eluted with a pH gradient such as that generated by a Pharmalyte 8-10.5 buffer (Pharmacia). Fractions were collected (8ml), aliquots diluted in PBS containing BSA (0.1 mg/ml) and prorelaxin detected by the enzyme linked immunosorbent assay (ELISA). A typical column profile is shown in figure 6. Fractions from the last eluting peak were pooled, dialysed against PBS using 6,000-8,000 dalton cut-off dialysis tubing (S&P), and analyzed by SDS-polyacrylamide gel electrophoresis. Gels stained with coomassie blue and gels blotted by the western immunoblot technique, using prorelaxin antibody described above, are shown in figure 7. Discrete protein bands were obtained, one of which reacted with the relaxin antibody. This protein band of approximate molecular weight 17,000 daltons was shown by protein sequence analysis (see below) to be prorelaxin.

Further purification of prorelaxin was achieved by gel sieving chromatography. The dialyzed pooled fractions were equilibrated using .01% Tween 20 and were concentrated using a YM10 filter membrane and a 10ml Amicon high-pressure concentration device. The concentrate (reduced in volume 20-30 fold) was centrifuged in 0.5ml aliquots for 2 minutes at 10,000g. The supernatants were injected onto an FPLC apparatus fitted with 2 10mm x 300mm Superose 12 columns (Pharmacia) connected in series and equilibrated with PBS - 0.01% Tween 20. The columns were run at 0.25 ml/minute and 0.25 ml fractions were collected. Eluting material was monitored by the absorbance at 280 nm (fig. 8a). Fractions were also assayed by ELISA (fig. 8b). The column resolved the protein species into well defined peaks. Peak #B was clearly identified by ELISA as being immunologically related to relaxin and was subsequently shown by amino acid sequence analysis (see below) to be prorelaxin. The pooled fractions from peak #3 were essentially free from detectable contaminants by gel electrophoresis at least 95% of the total protein based on densitometer scan of the gel was prorelaxin (fig. 9).

The identity of this peak as prorelaxin was confirmed by amino acid sequence analysis. Sequence analysis of recombinantly produced prorelaxin established that prorelaxin has an aspartic acid at the $NH_2$-terminus followed by a serine. Figure 11. This establishes for the first time the amino terminus of prorelaxin eliminating the speculation made using the DNA sequence. See e.g. Hudson et. al. EMBO Journal, supra. The claimed human prorelaxin of the instant invention comprises one hundred sixty-one amino acids with thirty-three of those amino acids in the B chain.

## Example 5
## Biological Assays

The human prorelaxin was tested in the murine pubic symphysis assay (MPS) using Charles River albino CFW female mice weighing 18-20 g., J. St. Louis, Can. J. Physiol. Pharmacol. 59, 507-512 (1981). Estradiol priming solution is 5 $\mu$g estradiol 17$\beta$-cyclopentylpropionate in 0.1 ml peanut oil. Synthetic relaxin ($H_2[B33A24]_BLys^3{}_BAla^{24}$) dose solutions are at concentrations of 0.62, 1.25, 2.50, 5.0, 10.0, 20.0, and 40.0 $\mu$g/ml in PBS - 0.01% Tween 20 - benzopurpurine 4B. Prorelaxin dose solutions are at 2.2, 4.4, 8.7, 17.5, 35.0, 70.0 and 140.0 $\mu$g/ml in the same buffer. When mice have been housed for at least six days in quarantine and weigh 18-20 g, each one is given a subcutaneous injection of estradiol priming solution. Exactly seven days later mice are injected subcutaneously with 0.2 ml of the appropriate human relaxin or prorelaxin dose solution. Between 18 and 20 hours after the human relaxin or prorelaxin injection the mice were sacrificed by cervical dislocation. The interpubic ligament is exposed and measured with a micrometer. These results are shown in Figure 10. Based on per mole of protein, relaxin and prorelaxin have similar activity in this assay.

Human prorelaxin's induction of cervical ripening in a non-human primate pregnancy will be tested. Human relaxin will be tested in time-mated Rhesus monkeys at gestational ages from 130 to 160 days. Term gestation in the colony is 168 ± 6 days. The animals will be adapted to wearing jackets with tethers. The tethered jackets will permit continuous access to indwelling catheters placed either in the femoral vessels or in the carotid and internal jugular vessels. In most animals a pressure transducer will be placed within the amniotic sac and electrodes connected to the uterine surface allowing measurement of uterine contractile activity. Prorelaxin will be administered as a continuous IV infusion over one hour, with blood samples obtained before, during and after the infusion at intervals from 15 minutes through the first hour following the infusion and then regularly for 24 hours. Doses of relaxin will range from 10 μg to 500 μg, and the cervix will be scored for texture, effacement, position, dilatation, and fetal position relative to the cervix, as well as the quality of the uterine lower segment. In most cases, two observers will examine each cervix and score them independently. Controls will be monkeys operated upon in similar manner and infused with saline (one) or monkeys maintained in the colony but not operated upon with cervical exams performed at weekly intervals.

### Example 6

### cAMP Uterine Cell Assay

Human gene 2 relaxin H2 (B33 A24) was used for the assay optimization and has subsequently been used as the assay standard. It was stored in a 0.1 mg/ml concentration at 4°C. A-chain H2 (A24) and B-chain H2 (B33) and purified porcine and rat prorelaxin were also tested in the assay. All relaxin dilutions were prepared with HAM Nutrient Mixture F12/Dulbecco's modified eagle medium (50:50) supplemented with 0.1% bovine serum albumin (F-12/DMEM/0.1% BSA) in polypropylene tubes.

The uterus of a newborn rhesus monkey delivered by caesarian section was stripped of mesentery, slit longitudinally, minced into 1mm pieces and washed three times with cold Hank's balanced salt solution (HBSS). The minced tissue was placed in a dissociating solution of 0.1% trypsin in HBSS with a magnetic stirrer for 1 hr. The suspension was centrifuged at 200g for 10 minutes, supernatant removed, and the cells resuspended in Eagle's minimum essential medium (EMEM) containing 10% FBS, non-essential amino acids, 10mM Hepes, 100 u/ml penicillin, 100 $\mu$g/ml streptomycin, and 20 u/ml mystatin. The cell preparation was filtered through sterile gauze then seeded in 6 wells of a Costar tissue culture cluster dish. The cultures were maintained at 37°C in an atmosphere of air (95%) and $CO_2$ (5%). Medium was changed 24 hr. after plating. A mixed cell population was observed microscopically with the myometrial smooth muscle cell type predominating and a small population of cells with epithelial-like morphology also present. Cultures were confluent after 9 days and were subcultured following trypsinization, using a split ratio of 1:2. With each successive passage, the percent of epithelial-like cells decreased and they were no longer evident microscopically at passage 4. At this point, the culture medium was changed to F-12/DMEM supplemented with L-glutamine 2mM, 10% FBS, 24mM hepes, 50 u/ml penicillin and 50$\mu$g/ml streptomycin (F-12/DMEM/10% FBS) and the split ratio increased to 1:3. Figure 3 is a phase contrast photomicrograph of cells produced according to the foregoing procedure (referred to as "NRMU" cells) at passage 5 showing the myometrial smooth muscle characteristics of fusiform cells with an oval or sausage-shaped nucleus growing in parallel arrays. Chamley-Campbell et al., Physiol. Rev. 59, 1-61 (1979). Casey et al., In Vitro 20, 396-403 (1984). The cells have been carried for 30 passages to date. Morphologically, they closely resemble the passage 5 cultured cells, however, occasional giant and multinucleated cells are apparent.

LC8x133.mhg

NRMU cells will be suspended in F-12/DMEM/10% FBS at 5 x $10^4$ cells/ml and will be seeded 2 ml/well into 6-well tissue culture plates (Falcon). Cells will be incubated at 37°C in 5% $CO_2$ for 18-24 hr. This will result in a 50-70% confluent monolayer at the time of assay. Spent medium will be removed and cells will be rapidly washed twice with 2 ml PBS/well. PBS will be removed and 0.5 ml of 0.1 mM IBMX added to each well. Plates will be covered and incubated at 37°C, 5% $CO_2$ for 30 min. Following this incubation, forskolin 4 μM and prorelaxin will be added simultaneously. Appropriate cell, forskolin and prorelaxin controls will be included in each assay. The final concentration of forskolin will be 1 μM/well. The plates will be rotated gently and immediately incubated at 37°C, 5% $CO_2$ for 15 min. Following incubation, lids will be removed and plates microwaved at the high setting for 1 minute to stop the biological reaction. Plates will be placed on ice and 0.5 ml of 0.2M sodium acetate will be added to each well and the plates rotated to wet the entire cell surface then covered and placed in a Revco freezer at -70°C for a minimum of 30 min. The cAMP assay (Amersham Corp., Arlington Heights, IL, Code TRK. 432) will be performed at room temperature after thawing the plates. The assay will be performed according to manufacturers' assay procedure.

Pharmaceutical Compositions

The compounds of the present invention can be formulated according to known methods to prepare pharmaceutically useful compositions, whereby the human prorelaxin is combined in a mixture with a pharmaceutically acceptable carrier vehicle. Suitable vehicles and their formulation, inclusive of other human proteins, e.g. human serum albumin, are described for example in Remington's Pharmaceutical Sciences by E.W. Martin, which is hereby incorporated by reference. Such compositions will contain an effective amount of the human prorelaxin protein hereof together with a suitable amount of vehicle in order to prepare pharmaceutically acceptable compositions suitable for effective administration to the host. For example, the human prorelaxin of the instant invention may be parenterally administered to pregnant subjects having difficulty delivering a term fetus; and treating certain connective tissue disorders such as scleroderma.

The average current dosage for the treatment of a problem pregnancy varies depending on numerous indicia known to the ordinarily skilled physician. The average doses administered intravenously are in the range of: 10 μg per kilogram to 1500 μg per kilogram, typically about 400 μg per kilogram, for effecting reproductive functions such as preparation of the birth canal with the dose varying depending upon the stage of gestation and condition of the patient and such other considerations as would be well known to the ordinarily skilled physician.

CLAIMS

1. Human prorelaxin essentially free of other naturally occurring human proteins.

2. A method comprising

a) transforming a eukaryotic host cell with an expression vector comprising nucleic acid encoding human prorelaxin under the control of transcriptional and translational control signals;

b) culturing the host cell of step a; and

c) recovering prorelaxin from the culture of step b.

3. An expression vector capable of expressing a DNA sequence encoding human prorelaxin in a host cell.

4. A host cell transformed with an expression vector of claim 3 for the expression of human prorelaxin.

5. The host cell of claim 4 that is a eukaryotic host cell for continuous recombinant expression of human prorelaxin essentially free of other naturally occurring human proteins.

6. The eukaryotic host cell of claim 5 that is human embryonic kidney cell (293).

7. The eukaroytic host cell of claim 5 that is Mouse sertoli cell (TM4)

8. A composition comprising human prorelaxin.

9. A sterile therapeutic composition comprising a therapeutically effective amount of human prorelaxin in a pharmacologically suitable carrier.

10. Human prorelaxin for therapeutic use.

11. Human prorelaxin for use in effecting parturition in an animal.

12. Human prorelaxin for use in treating connective tissue disorders in an animal.

13. The use of human prorelaxin in the manufacture of a medicament for effecting parturition or for treating connective tissue disorders.

AT, GR, ES

CLAIMS

1. A method which comprises the preparation of human prorelaxin essentially free of other naturally occurring human proteins.

2. A method comprising

a) transforming a eukaryotic host cell with an expression vector comprising nucleic acid encoding human prorelaxin under the control of transcriptional and translational control signals;

b) culturing the host cell of step a; and

c) recovering prorelaxin from the culture of step b.

3. A method which comprises the preparation of an expression vector capable of expressing a DNA sequence encoding human prorelaxin in a host cell.

4. A host cell transformed with an expression vector of claim 3 for the expression of human prorelaxin.

5. The host cell of claim 4 that is a eukaryotic host cell for continuous recombinant expression of human prorelaxin essentially free of other naturally occurring human proteins.

6. The eukaryotic host cell of claim 5 that is human embryonic kidney cell (293).

7. The eukaroytic host cell of claim 5 that is Mouse sertoli cell (TM4).

8. The use of human prorelaxin in the manufacture of a medicament.

9. The use of human prorelaxin in the manufacture of a medicament for effecting parturition or for treating connective tissue disorders.

Fig.1.

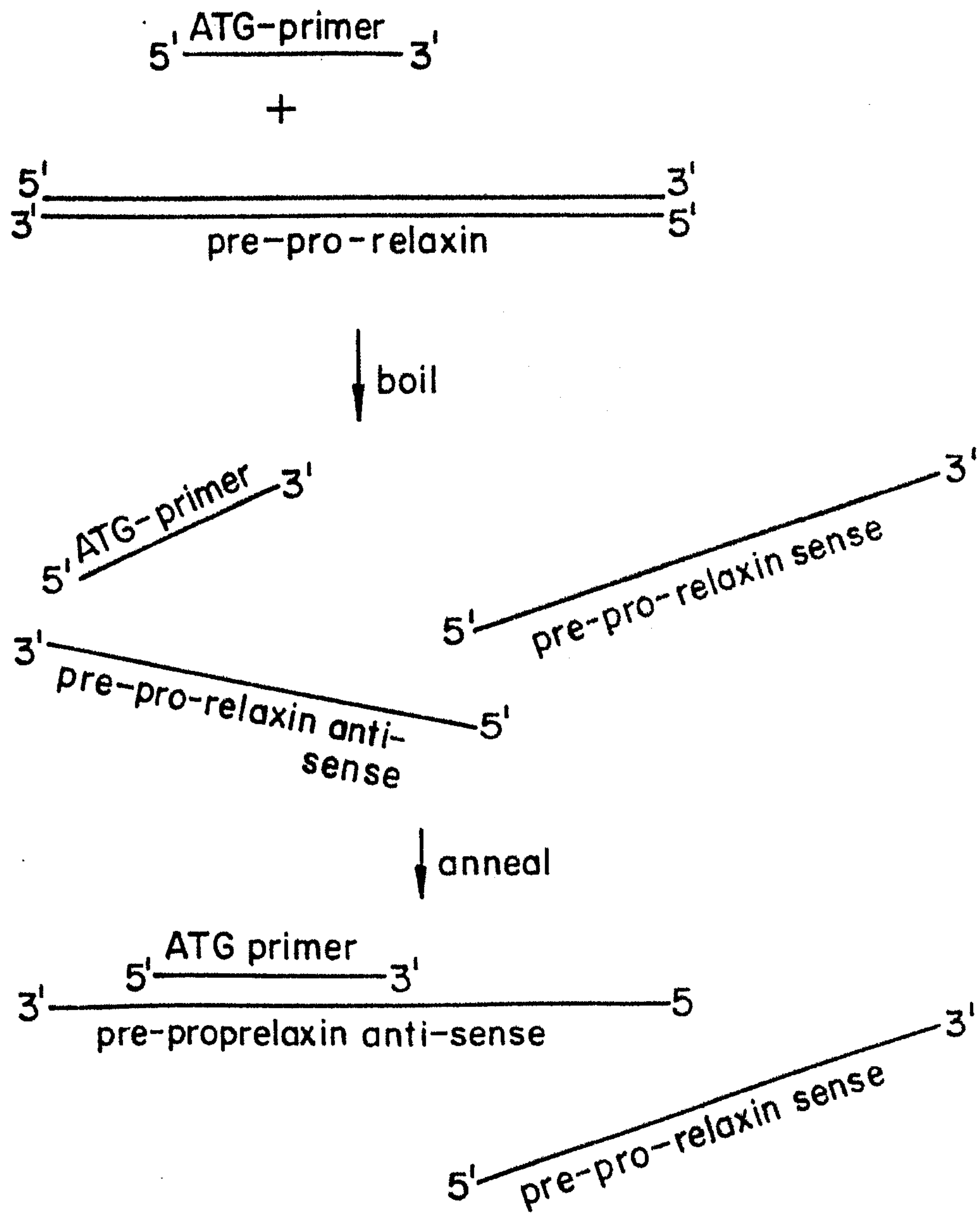
5' ATG-primer 3'
+
5' 3'
3' 5'
pre-pro-relaxin
boil
5' ATG-primer 3'
5' pre-pro-relaxin sense 3'
3' pre-pro-relaxin anti-sense 5'
anneal
ATG primer
5' 3'
3' 5
pre-proprelaxin anti-sense
5' pre-pro-relaxin sense 3'

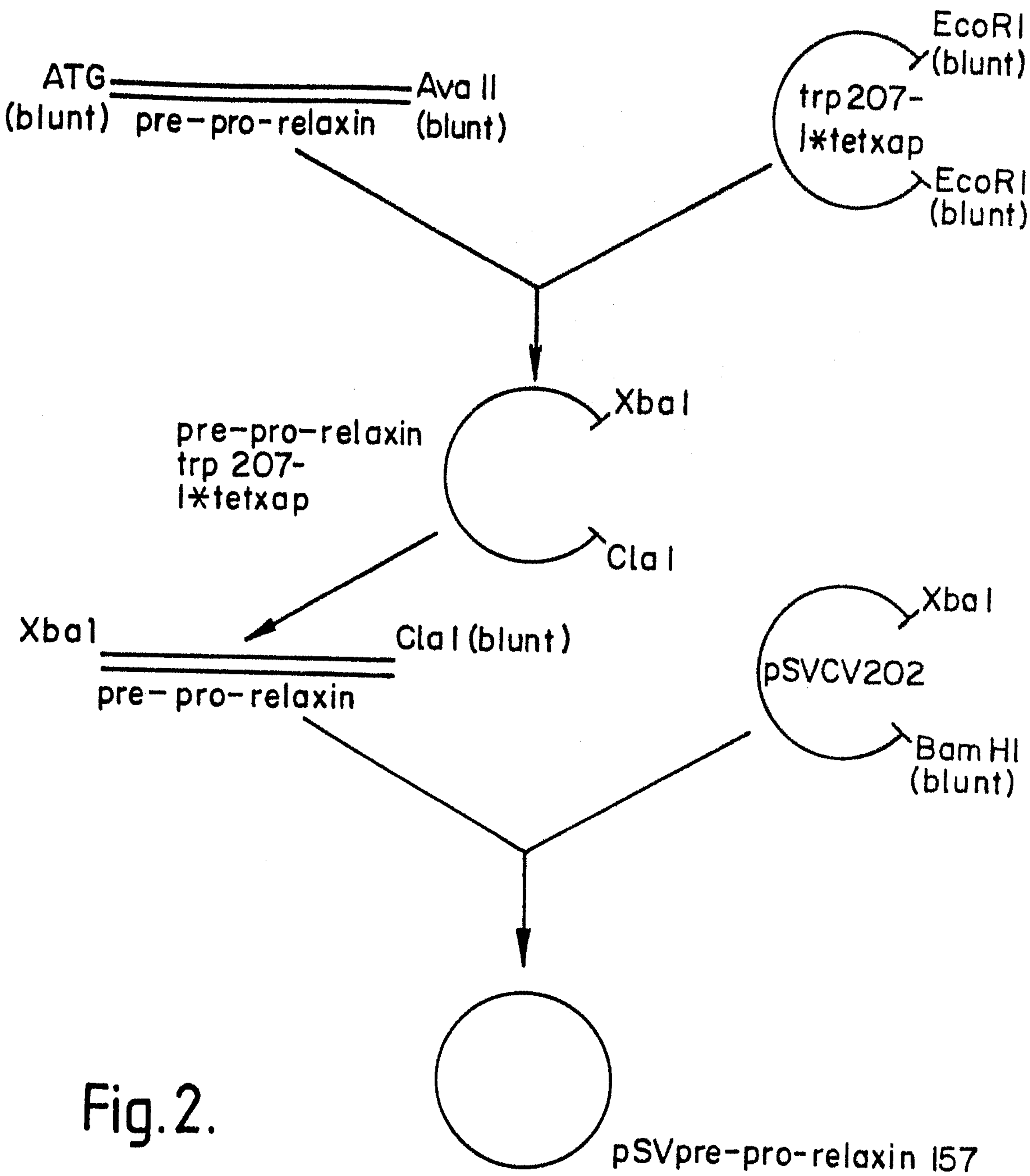
ATG
(blunt)
pre-pro-relaxin
Ava II
(blunt)
EcoRI
(blunt)
trp 207-
I*tetxap
EcoRI
(blunt)
pre-pro-relaxin
trp 207-
I*tetxap
XbaI
ClaI
XbaI
ClaI (blunt)
pre-pro-relaxin
XbaI
pSVCV202
Bam HI
(blunt)
pSVpre-pro-relaxin 157

Fig. 2.

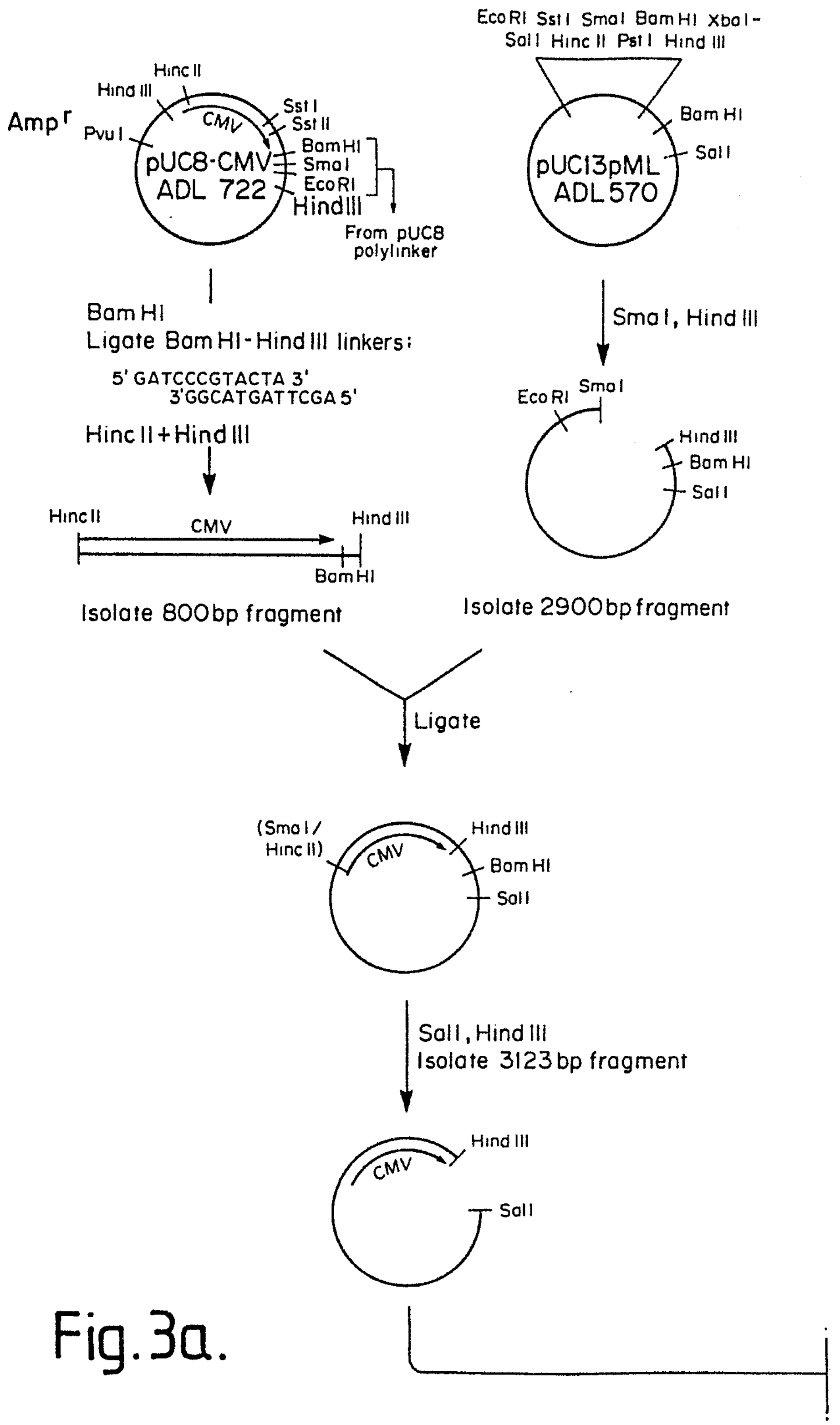
Amp r
Pvu I
Hind III
Hinc II
CMV
Sst I
Sst II
pUC8-CMV
ADL 722
Bam HI
Sma I
EcoRI
HindIII
From pUC8
polylinker
Bam HI
Ligate Bam HI-Hind III linkers:
5' GATCCCGTACTA 3'
3'GGCATGATTCGA 5'
Hinc II+Hind III
Hinc II
CMV
Hind III
Bam HI
Isolate 800bp fragment
EcoRI SstI SmaI BamHI XbaI-
SalI Hinc II PstI Hind III
Bam HI
Sal I
pUC13pML
ADL 570
Sma I, Hind III
Eco RI
Sma I
Hind III
Bam HI
Sal I
Isolate 2900bp fragment
Ligate
(Sma I/
Hinc II)
CMV
Hind III
Bam HI
Sal I
Sal I, Hind III
Isolate 3123bp fragment
CMV
Hind III
Sal I

Fig.3a.

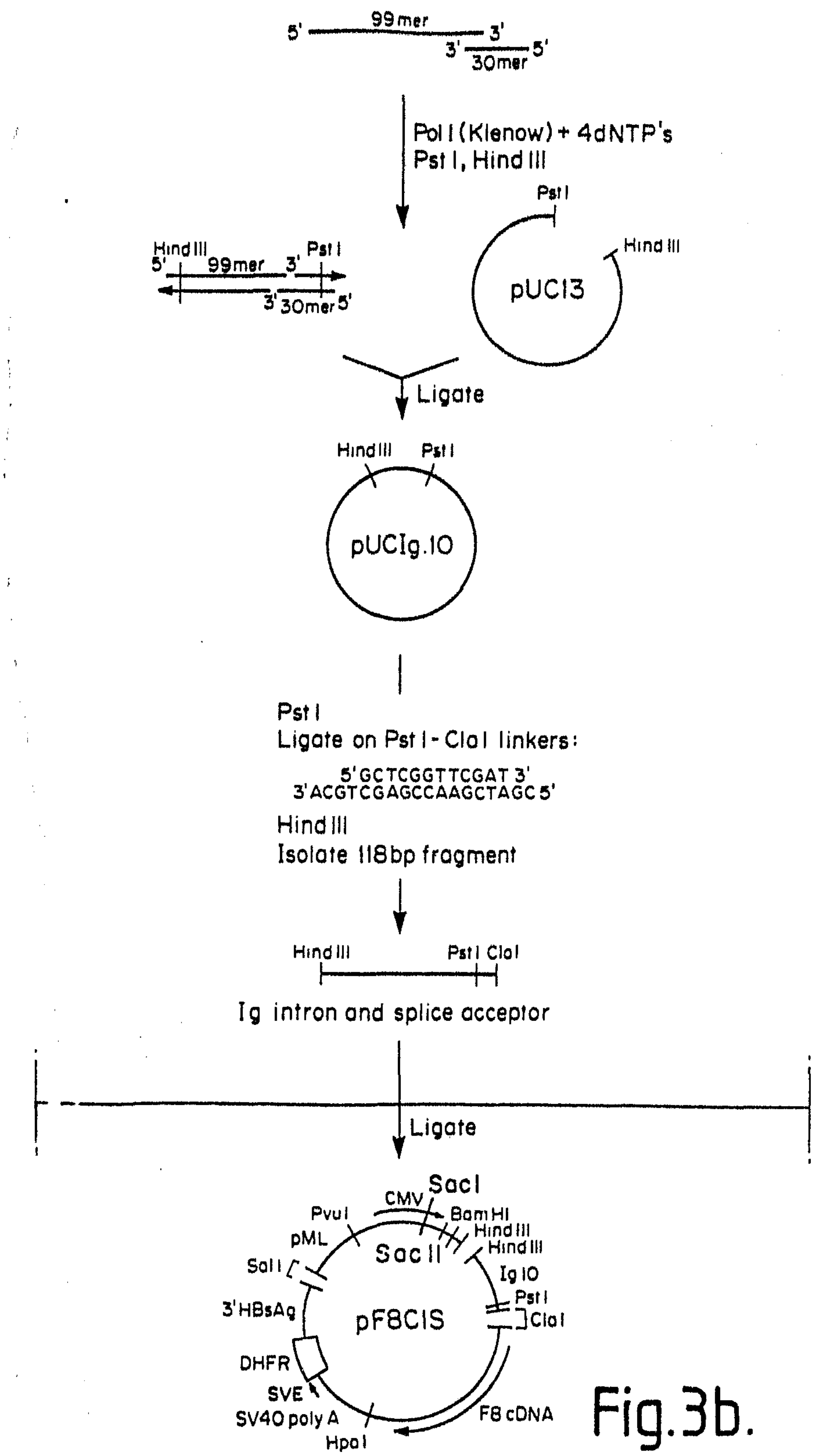
5' 99mer 3'
3' 30mer 5'
Pol I (Klenow) + 4dNTP's
Pst I, Hind III
Hind III 5' 99mer 3' Pst I
3' 30mer 5'
Pst I
Hind III
pUC13
Ligate
Hind III Pst I
pUCIg.10
Pst I
Ligate on Pst I - Cla I linkers:
5'GCTCGGTTCGAT 3'
3'ACGTCGAGCCAAGCTAGC 5'
Hind III
Isolate 118bp fragment
Hind III Pst I Cla I
Ig intron and splice acceptor
Ligate
Sac I
CMV
Pvu I
Bam HI
Hind III
Hind III
pML
Sac II
Sal I
Ig 10
Pst I
Cla I
3'HBsAg
pF8CIS
DHFR
SVE
SV40 poly A
Hpa I
F8 cDNA

Fig.3b.

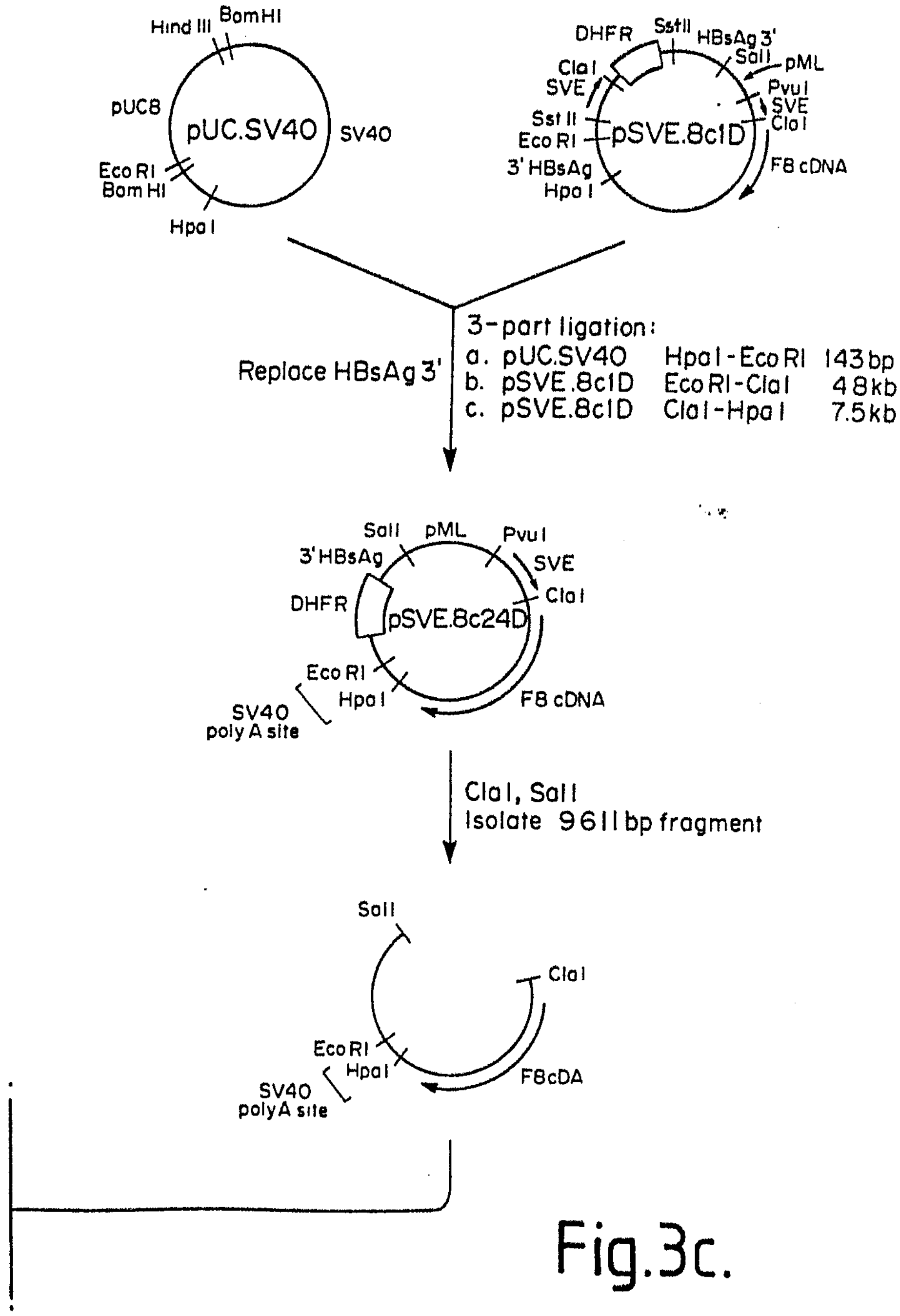
Hind III
BamHI
pUC8
pUC.SV40
SV40
Eco RI
Bam HI
Hpa I
DHFR
SstII
HBsAg 3'
Cla I
SVE
SalI
pML
Pvu I
SVE
Cla I
Sst II
Eco RI
pSVE.8c1D
3' HBsAg
Hpa I
F8 cDNA
Replace HBsAg 3'
3-part ligation:
a. pUC.SV40 HpaI-EcoRI 143bp
b. pSVE.8c1D EcoRI-ClaI 48kb
c. pSVE.8c1D ClaI-HpaI 7.5kb
SalI
pML
Pvu I
3'HBsAg
SVE
DHFR
Cla I
pSVE.8c24D
Eco RI
Hpa I
F8 cDNA
SV40
poly A site
ClaI, SalI
Isolate 9611 bp fragment
SalI
ClaI
Eco RI
Hpa I
F8cDA
SV40
polyA site

Fig.3c.

Fig.4.

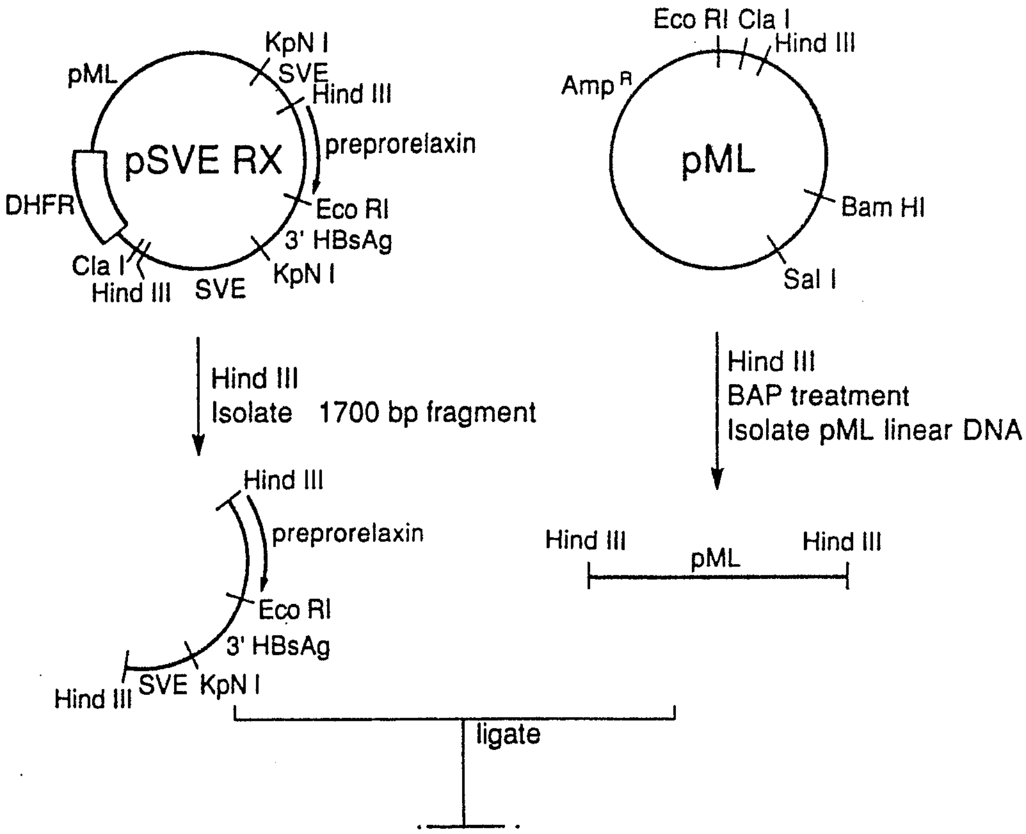
pML
KpN I
SVE
Hind III
pSVE RX
preprorelaxin
DHFR
Eco RI
3' HBsAg
Cla I
Hind III
SVE
KpN I
Eco RI Cla I
Hind III
Amp R
pML
Bam HI
Sal I
Hind III
Isolate 1700 bp fragment
Hind III
BAP treatment
Isolate pML linear DNA
Hind III
preprorelaxin
Eco RI
3' HBsAg
Hind III
SVE
KpN I
Hind III
pML
Hind III
ligate

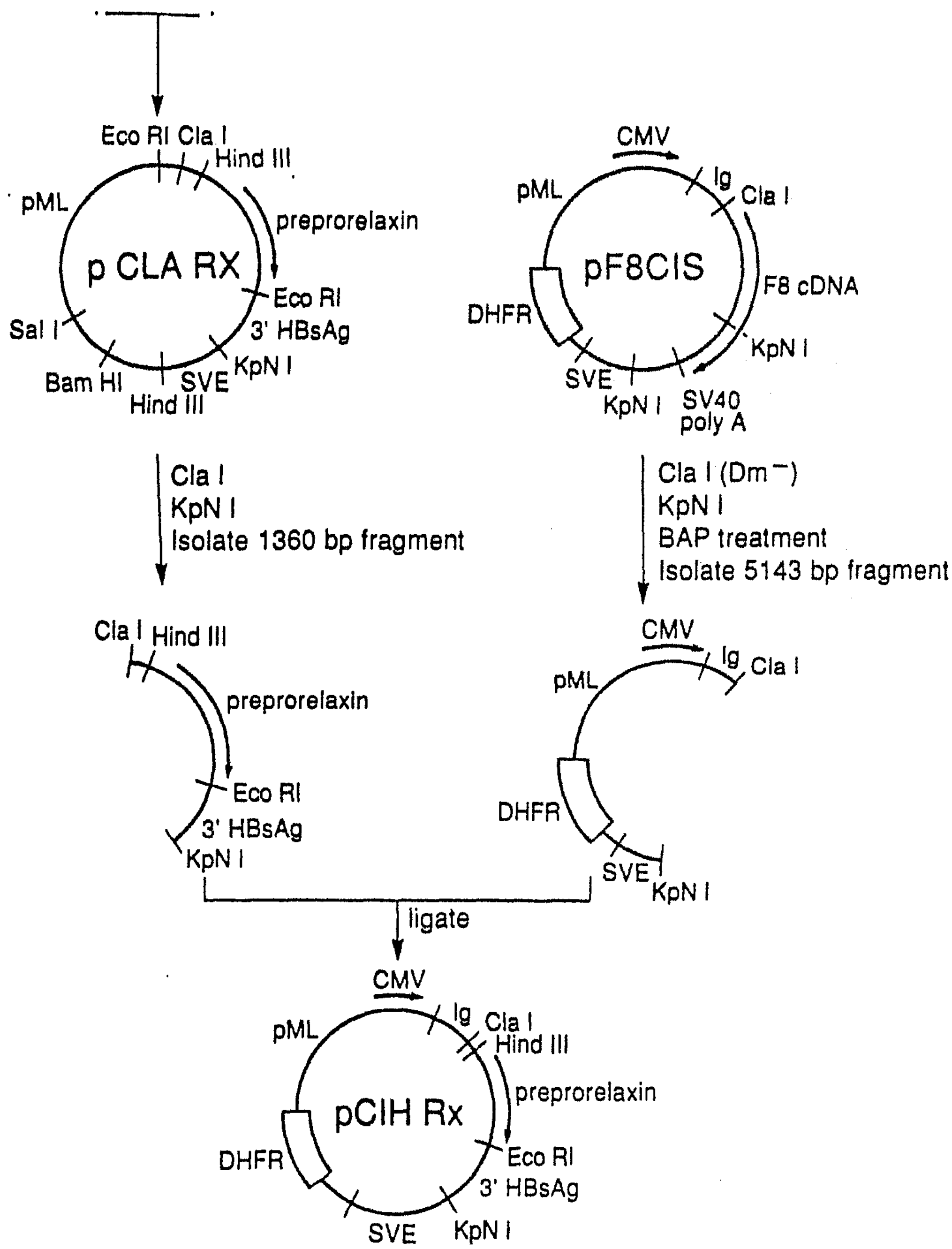

Eco RI Cla I
Hind III
pML
preprorelaxin
p CLA RX
Eco RI
Sal I
3' HBsAg
KpN I
Bam HI
SVE
Hind III
CMV
pML
Ig
Cla I
pF8CIS
F8 cDNA
DHFR
KpN I
SVE
KpN I
SV40
poly A
Cla I
KpN I
Isolate 1360 bp fragment
Cla I (Dm⁻)
KpN I
BAP treatment
Isolate 5143 bp fragment
Cla I
Hind III
preprorelaxin
Eco RI
3' HBsAg
KpN I
CMV
pML
Ig
Cla I
DHFR
SVE
KpN I
ligate
CMV
pML
Ig
Cla I
Hind III
preprorelaxin
pCIH Rx
DHFR
Eco RI
3' HBsAg
SVE
KpN I


Fig.4(cont.)

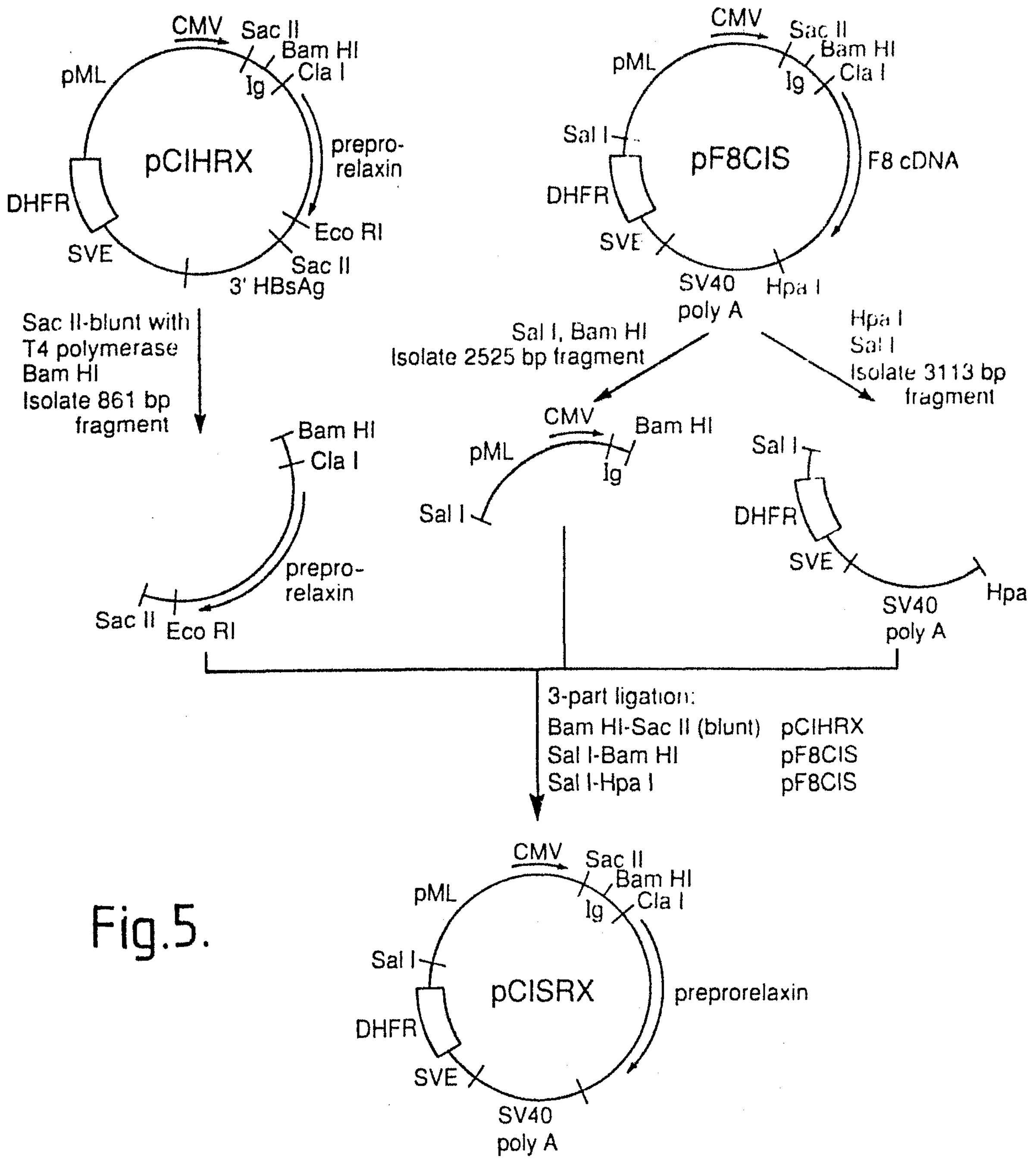


Fig.5.

BAD ORIGINAL

Fig.6.

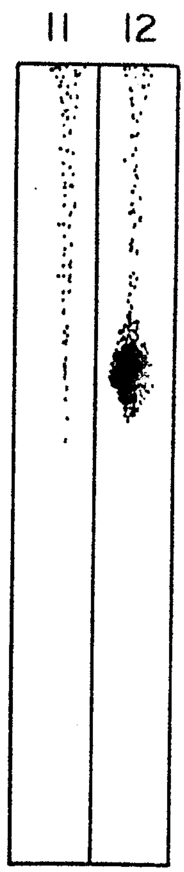

Fig.7.

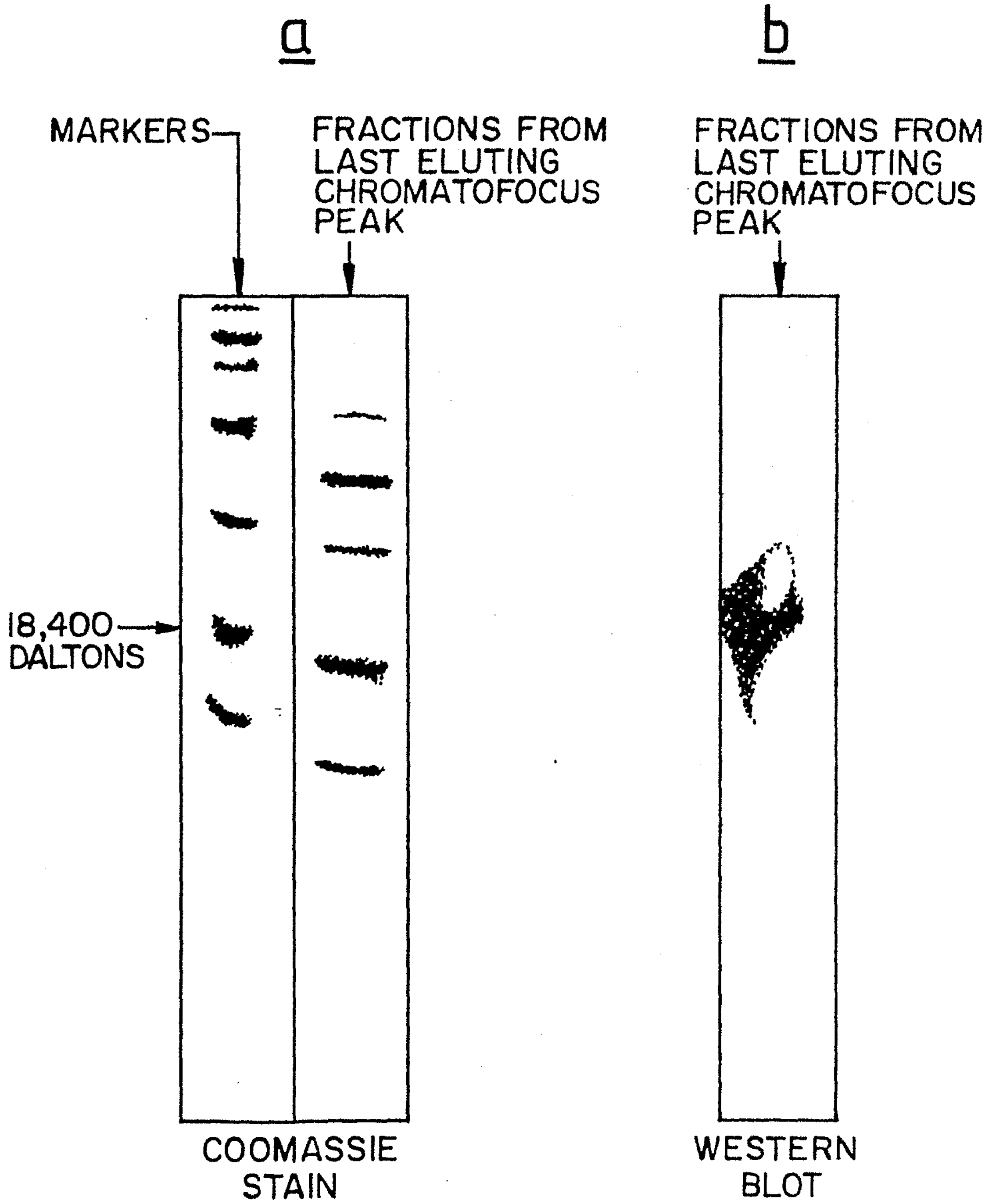
a
b
MARKERS
FRACTIONS FROM LAST ELUTING CHROMATOFOCUS PEAK
FRACTIONS FROM LAST ELUTING CHROMATOFOCUS PEAK
18,400 DALTONS
COOMASSIE STAIN
WESTERN BLOT

Fig.8a.

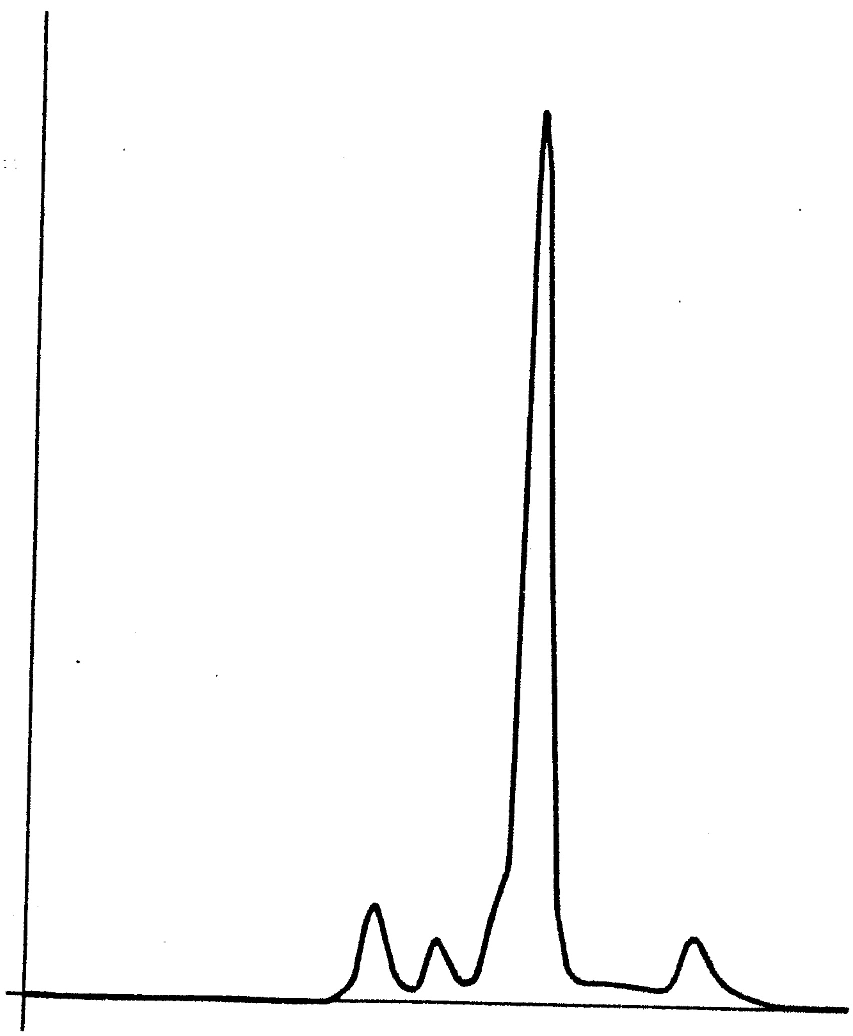

Fig. 8b.

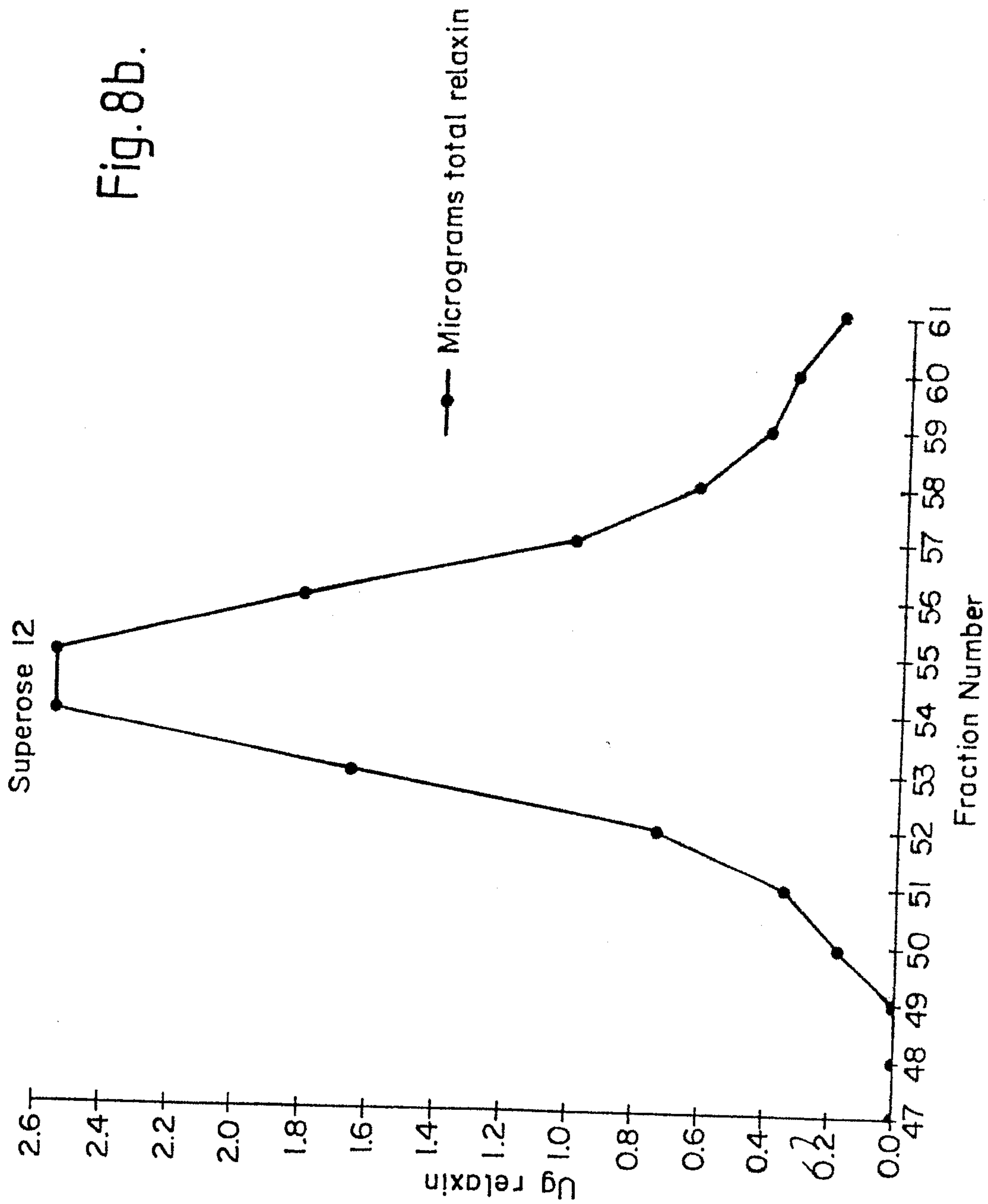
Superose 12
Micrograms total relaxin
Ug relaxin
2.6
2.4
2.2
2.0
1.8
1.6
1.4
1.2
1.0
0.8
0.6
0.4
0.2
0.0
Fraction Number
47
48
49
50
51
52
53
54
55
56
57
58
59
60
61

Fig.9.

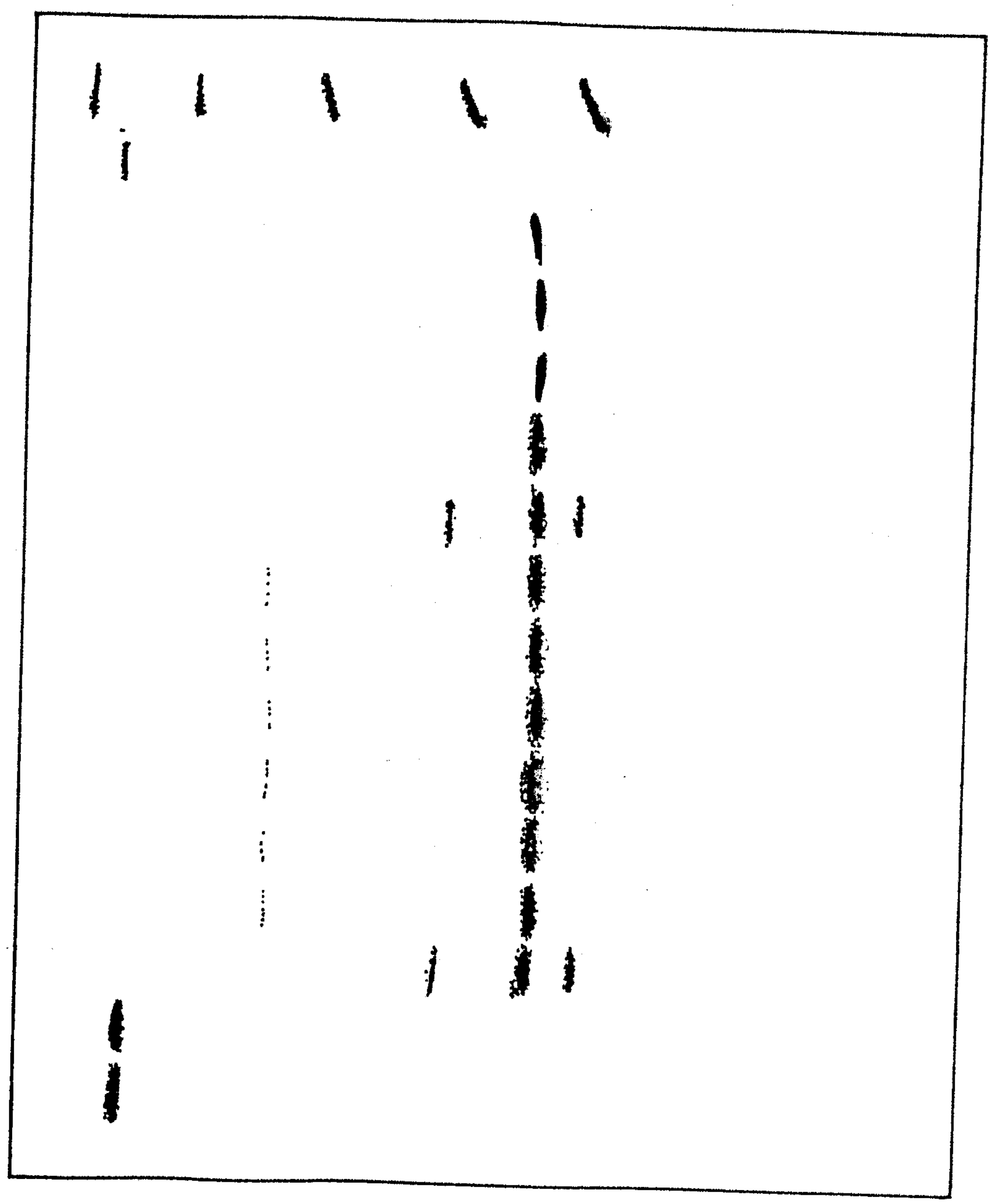

# Fig.10.

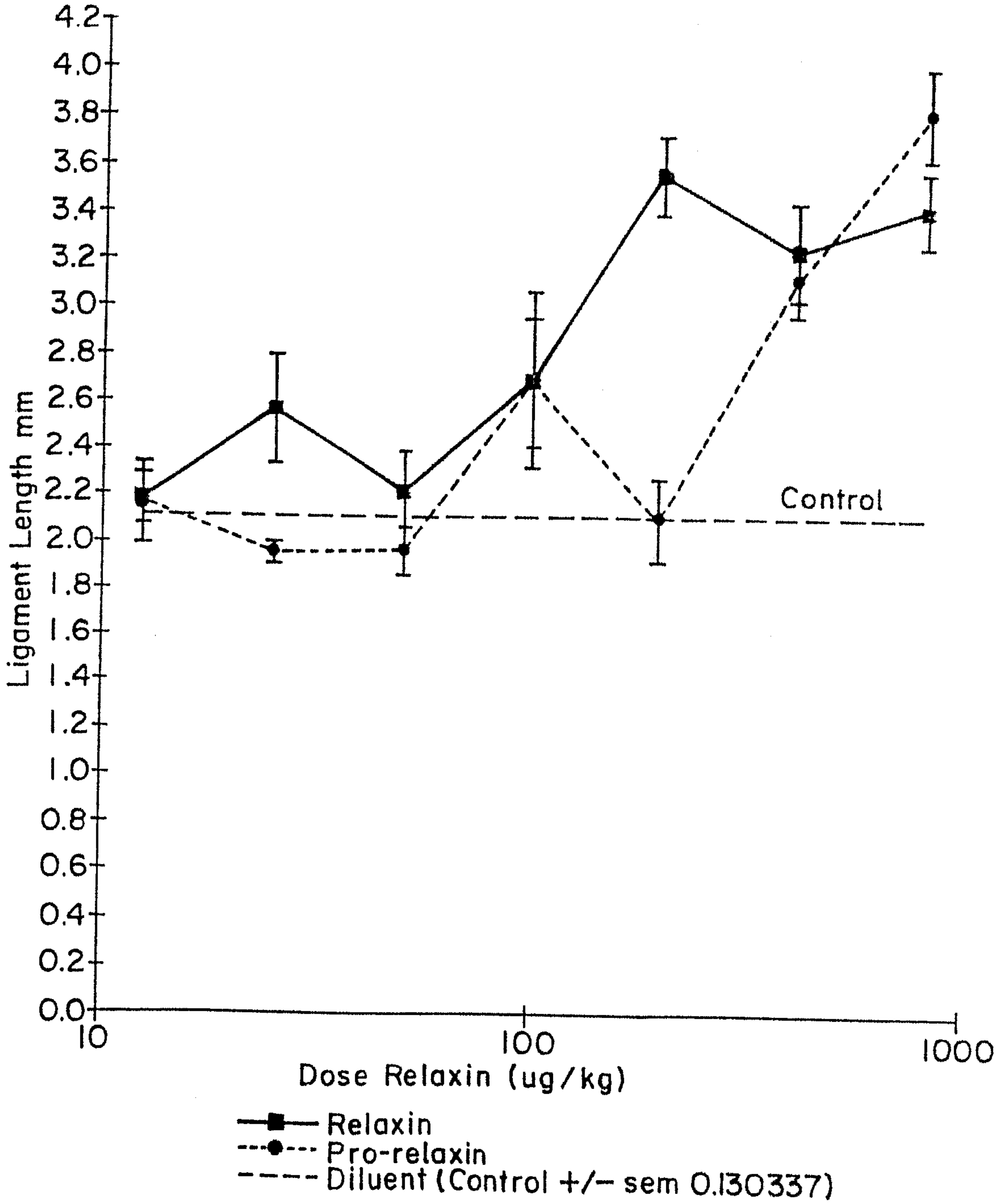
4.2
4.0
3.8
3.6
3.4
3.2
3.0
2.8
2.6
2.4
2.2
2.0
1.8
1.6
1.4
1.2
1.0
0.8
0.6
0.4
0.2
0.0
Ligament Length mm
Control
10
100
1000
Dose Relaxin (ug/kg)
Relaxin
Pro-relaxin
Diluent (Control +/- sem 0.130337)

TCTAGAATT ATG CCT CGC CTG TTT TTT TTC CAC CTG CTA GGA GTC TGT TTA CTA CTG AAC CAA TTT TCC AGA GCA GTC GCG
Met Pro Arg Leu Phe Phe Phe His Leu Leu Gly Val Cys Leu Leu Leu Asn Gln Phe Ser Arg Ala Val Ala

B →
GAC TCA TGG ATG GAG GAA GTT ATT AAA TTA TGC GGC CGC GAA TTA GTT CGC GCG CAG ATT GCC ATT TGC GGC ATG AGC ACC
1 Asp Ser Trp Met Glu Glu Val Ile Lys Leu Cys Gly Arg Glu Leu Val Arg Ala Gln Ile Ala Ile Cys Gly Met Ser Thr

33 C →
TGG AGC AAA AGG TCT CTG | AGC CAG GAA GAT GCT CTT CAG ACA CCT AGA CCA GTG GCA GAA ATT GTG CCA TCC TTC ATC AAC
28 Trp Ser Lys Arg Ser Leu | Ser Gln Glu Asp Ala Pro Gln Thr Pro Arg Pro Val Ala Glu Ile Val Pro Ser Phe Ile Asn

AAA GAT ACA GAA ACC ATA AAT ATG ATG TCA GAA TTT GTT GCT AAT TTG CCA CAG GAG CTG AAG TTA ACC CTG TCT GAG ATG
55 Lys Asp Thr Glu Thr Ile Asn Met Met Ser Glu Phe Val Ala Asn Leu Pro Gln Glu leu Lys Leu Thr leu Ser Glu Met

CAG CCA GCA TTA CCA CAG CTA CAA CAA CAT GTA CCT GTA TTA AAA GAT TCC AGT CTT CTC TTT GAA GAA TTT AAG AAA CTT
82 Gln Pro Ala Leu Pro Gln Leu Gln Gln His Val Pro Val Leu Lys Asp Ser Ser Leu Leu Phe Glu Glu Phe Lys Lys Leu

ATT CGC AAT AGA CAA AGT GAA GCC GCA GAC AGC AGT CCT TCA GAA TTA AAA TAC TTA GGC TTG GAT ACT CAT TCT CGA AAA
109 Ile Arg Asn Arg Gln Ser Glu Ala Ala Asp Ser Ser Pro Ser Glu Leu Lys Tyr Leu Gly Leu Asp Thr His Ser Arg Lys

← C A →
AAG AGA | CAA CTC TAC AGT GCA TTG GCT AAT AAA TGT TGC CAT GTT GGT TGT ACC AAA AGA TGT CTT GCT AGA TTT TGC TGA
136 Lys Arg | Gln Leu Tyr Ser Ala Leu Ala Asn Lys Cys Cys His Val Gly Cys Thr Lys Arg Ser Leu Ala Arg Phe Cys OP*

Fig.11.

European Patent Office

# EUROPEAN SEARCH REPORT

0260149

Application Number

EP 87 30 8062

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 112 149 (HOWARD FLOREY INSTITUTE)<br>* Page 17, lines 1-11; page 15, lines 1-19 *<br>--- | 1,2-5 | C 12 N 15/00<br>C 12 P 21/02<br>C 07 K 7/10<br>C 12 N 5/00<br>A 61 K 37/24 |
| A | US-A-4 267 101 (M. BIGAZZI)<br>* Column 1, lines 64-67 *<br>--- | 8-13 | |
| A,P | WO-A-8 605 810 (BIOGEN)<br>* Claims 3-5 *<br>----- | 1-5 | |
| | | | TECHNICAL FIELDS SEARCHED (Int. Cl.4) |
| | | | C 12 N<br>A 61 K<br>C 07 K |

The present search report has been drawn up for all claims

| Place of search | Date of completion of the search | Examiner |
|---|---|---|
| THE HAGUE | 26-11-1987 | HUBER-MACK A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

BAD ORIGINAL